# FASCICULE DE BREVET EUROPEEN

(11) **EP 1 192 253 B1**
(45) Date de publication et mention de la délivrance du brevet: **25.01.2012**
(21) Numéro de dépôt: 00945990.0
(22) Date de dépôt: 22.06.2000
(51) Int. Cl.: C12N 15/12, C07K 14/435, C07K 14/47, C12N 15/10, C12N 15/66, C12N 15/11, C12Q 1/68, C07K 16/18, G01N 33/53, A61K 51/00, A61P 35/00

(54) **POLYPEPTIDE ICBP90 ET SES FRAGMENTS ET POLYNUCLEOTIDES CODANT LESDITS POLYPEPTIDES ET APPLICATIONS AU DIAGNOSTIC ET AU TRAITEMENT DU CANCER**
ICBP90-POLYPEPTIDE, POLYPEPTIDFRAGMENTE DAVON, DIE DAFÜR KODIERENDEN POLYNUKLEOTIDE, UND IHRE VERWENDUNG ZUR BEHANDLUNG UND DIAGNOSE VON KREBS
ICBP90 POLYPEPTIDE AND ITS FRAGMENTS AND POLYNUCLEOTIDES CODING FOR SAID POLYPEPTIDES AND APPLICATIONS FOR DIAGNOSING AND TREATING CANCER

(30) Priorité: 22.06.1999 FR 9907935
(43) Date de publication de la demande: 03.04.2002
(73) Titulaire: INSTITUT NATIONAL DE LA SANTE ET DE LA RECHERCHE MEDICALE (INSERM), 75654 Paris Cédex 13 (FR)
(72) Inventeur: BRONNER, Christian, F-67640 Fegersheim (FR); HOPFNER, Rapha¬l, F-67000 Strasbourg (FR); MOUSLI, Marc, F-67400 Illkirch (FR); JELTSCH, Jean-Marc, F-67120 Molsheim (FR); LUTZ, Yves, F-67000 Strasbourg (FR); OUDET, Pierre, F-67000 Strasbourg (FR)
(74) Mandataire: Lecocq, Fabrice
(86) Numéro de dépôt international: PCT/FR2000/001747
(87) Numéro de publication internationale: WO 2000/078949

(56) Documents cités:
- WO-A-98/37207
- WO-A-99/38972
- DATABASE EMBL NUCLEOTIDE AND PROTEIN SEQUENCES, 18 août 1998 (1998-08-18), XP002133353 HINXTON, GB
- DATABASE EMBL NUCLEOTIDE AND PROTEIN SEQUENCES, 18 avril 1997 (1997-04-18), XP002133354 HINXTON, GB
- FUJIMORI A. ET AL.,: "Cloning and mapping of Np95 gene which encodes a novel nuclear protein associated with cell proliferation." MAMMALIAN GENOME, vol. 9, no. 12, 1998, page 1032-1035 XP000890078
- SANDRI M I ET AL: "P53 REGULATES THE MINIMAL PROMOTER OF THE HUMAN TOPOISOMERASE IIALPHA GENE" NUCLEIC ACIDS RESEARCH,GB,OXFORD UNIVERSITY PRESS, SURREY, vol. 24, no. 22, 15 novembre 1996 (1996-11-15), pages 4464-4470, XP002068824 ISSN: 0305-1048 cité dans la demande
- ISAACS RJ T AL.,: "Regulation of the human topoisomerase IIalpha gene promoter in confluence arrested cells." THE JOURNAL OF BIOLOGICAL CHEMISTRY, vol. 271, no. 28, 12 juillet 1996 (1996-07-12), pages 16741-16747, XP002133355 cité dans la demande
- HERZOG C E AND ZWELLING L A: "Evaluation of a potential regulatory role for inverted CCAAT boxes in the human topoisomerase IIalpha promoter" BIOCHEMICAL AND BIOPHYSICAL RESEARCH COMMUNICATIONS, vol. 232, 1997, pages 608-612, XP000877157 cité dans la demande
- LIM K ET AL.: "Reduced level of ATF is corelated with transcriptional repression of DNA topoisomerase IIalpha gene during TPA-induced differentiationof HL-60 cells" BIOCHEMISTRY AND MOLECULAR BIOLOGY INTERNATIONAL, vol. 46, no. 1, septembre 1998 (1998-09), pages 35-42, XP000900088 cité dans la demande
- KUBO T ET AL.,: "DNA topoisomerase IIalpha gene expression under transcriptional control in etoposide/teniposide-resistant human cancer cells" CANCER RESEARCH, vol. 55, 1 septembre 1995 (1995-09-01), pages 3860-3864, XP000877419 cité dans la demande
- DATABASE EMBL NUCLEOTIDE AND PROTEIN SEQUENCES, 3 avril 2000 (2000-04-03), XP002148667 HINXTON, GB
- HOPFNER R ET AL.,: "ICBP90, a novel human CCAAT binding protein, involved in the regulation of topoisomerase IIalpha expression." CANCER RESEARCH, vol. 60 (1), 1 janvier 2000 (2000-01-01), page 121-8 XP000884566

## Description

La présente invention concerne un nouveau polypeptide ICBP90 et ses fragments, le clonage de l'ADNc et les polynucléotides codant pour lesdits polypeptides, des vecteurs de clonage et/ou d'expression incluant lesdits polynucléotides, des cellules transformées par lesdits vecteurs et des anticorps spécifiques dirigés contre lesdits polypeptides. L'invention concerne également des procédés et des kits de diagnostic des cancers, un procédé et un kit de criblage de ligands du polypeptide de l'invention et des composés utilisables à titre de médicament pour la prévention et/ou le traitement des cancers.

Les ADN topoisomérases sont des protéines nucléaires hautement conservées au cours l'évolution dont le rôle principal est de contrôler la conformation et la topologie de l'ADN dans le noyau, qui sont constamment altérées par les différents processus biologiques impliquant l'ADN tels par exemple la transcription et la réplication. Les topoisomérases exercent leur action en coupant l'ADN et en reliant ces lésions après avoir réalisé le changement conformationnel adéquat.

Chez les mammifères et l'homme en particulier, il existe à l'heure actuelle au moins cinq gènes différents codant pour une topoisomérase et au moins deux pseudogènes additionnels (pour revue, voir Nitiss 1998). Ainsi, la topoisomérase I, codée par le gène TOP1 retire les supertours présents dans l'ADN en ne coupant qu'un seul brin. Les deux topoisomérases de type II existant chez l'homme appelées TopIIα et TopIIβ, altèrent la topologie de l'ADN en introduisant des clivages double brin transitoires (pour revue, voir Wang 1996). Enfin, il existe deux topoisomérases de type III codées par deux gènes localisés en 17p 11.2-12 et 22q 11-12 et qui agissent uniquement contre les supertours négatifs de l'ADN.

Dans les cellules tumorales, les topoisomérases de type II jouent un rôle très important ; dans ces cellules en croissance et en division rapide, il existe un grand besoin de maintenir les molécules d'ADN dans une conformation correcte puisque des taux de transcription et de réplication élevés sont nécessaires. Ainsi, les taux de toposiomérases II sont en général plus élevés dans les cellules tumorales humaines que dans les tissus normaux de même origine. Cependant, le taux d'expression élevé de la topoisomérase IIα dans les cellules tumorales peut variés entre deux tumeurs de nature différente affectant un même tissu. Par exemple, le noyau des cellules de carcinome du poumon à petites cellules présente un taux plus élevé de topoisomérase IIα que le noyau des cellules de carcinomes pulmonaires à cellules de taille normale (Guinee *et al*., 1996). De la même manière, le taux de topoisomérase IIα dans les cellules A549 est trois fois plus élevé que dans les cellules PC3, ces deux lignées cellulaires provenant d'adénocarcinome de l'épithélium pulmonaire (Yamasaki *et al*., 1996).

Ces constatations donnent à penser que la topoisomérase IIα peut être considérée comme un marqueur de prolifération cellulaire pour certains types de cancer. Le processus cancéreux se caractérisant par une prolifération cellulaire anormale due en partie à la perte de l'inhibition de contact, la topoisomérase IIα apparaît donc comme une cible privilégiée des drogues chimiothérapeutiques pour le traitement du cancer (Pommier *et al*. 1994), et les traitements anticancéreux actuels font largement appel aux inhibiteurs de topoisomérases.

La plupart de ces inhibiteurs exercent leurs effets cytotoxiques en stabilisant le complexe de clivage de l'ADN. Des drogues comme les anthracyclines [doxorubicine (adriamycine) ou épipodophyllotoxines (tel l'étoposide (VP-16) ou le téniposide (VM26))], les acridines (tel que le mAMSA) et les anthracendiones (e.g. mitoxantrone) sont des exemples de drogues inhibitrices de topoisomérases II qui stabilisent le complexe de clivage. Plus récemment, une nouvelle classe d'inhibiteurs de topoisomérases II a été développée ; ces inhibiteurs agissent au niveau de l'activité catalytique et non plus en stabilisant le complexe de clivage. La drogue fostriécine en est un exemple (Boritzki *et al*., 1988). Aujourd'hui ces différentes drogues sont utilisées dans des traitements anti-cancéreux curatifs et palliatifs.

Néanmoins, l'un des problèmes majeurs rencontré dans les traitements anti-cancéreux actuels utilisant les inhibiteurs des topoisomérases est l'émergence d'une résistance aux drogues (Kubo *et al*., 1995). Ces résistances sont soit le fait d'une surexpression de pompes permettant l'efflux de drogues à l'extérieur de cellules avant qu'elles n'atteignent leur cible (e.g ; P-glycoprotéine, protéine associée à la multirésistance aux drogues (MRP)), soit le fait de changement du taux d'expression de la topoisomérase IIα (Deffie *et al*., 1989; Fry *et al*., 1991), soit des deux (pour revue, voir Isaacs *et al*., 1998).

L'un des aspects de la présente invention est donc de comprendre les mécanismes de régulation de l'expression du gène de la topoisomérase IIα, afin de développer une alternative au phénomène de résistance aux drogues observé pour certains cancers, et ce, dans l'optique d'améliorer le traitement préventif et curatif des cancers.

Il existe deux types de topoisomérases de type II qui diffèrent dans leur profil d'expression ; la topoisomérase IIα (Top IIα) (170 kD), essentiellement localisée dans le nucléoplasme au niveau du centromère des chromosomes mitotiques, intervient dans les processus biologiques fondamentaux que sont la réplication, la condensation des chromosomes et la transcription. La topoisomérase IIβ (Top IIβ) (180 kD) est semble-t-il plutôt impliquée dans la transcription des ARN ribosomiques étant donné la localisation nucléolaire de cette enzyme. Les deux topoisomérases de type II humaines sont localisées sur deux chromosomes différents (17q21-22 pour la topoisomérase IIα et 3p24 pour la topoisomérase IIβ) (Tsai-Plugfelder *et al*., 1988 ; Drake *et al*., 1989 ; Chung *et al*., 1989 ; Jenkins *et al*., 1992 ; Austin *et al*., 1993).

Contrairement à la topoisomérase IIβ dont l'expression se caractérise par une relative constance, la topoisomérase IIα présente une variation d'expression en fonction de l'état de prolifération des cellules et de leur position dans le cycle cellulaire. L'expression de l'ARN messager (ARNm) est plus élevée dans les cellules en prolifération que dans les cellules arrêtées en confluence. L'expression de la topoisomérase IIα augmente au cours de la phase S du cycle cellulaire pour atteindre un maximum en fin de phase G2/M (Goswami *et al*., 1996), le niveau d'ARN messager étant dix fois plus élevé en fin de phase S que pendant la phase G 1. Egalement, il semble exister un couplage entre la synthèse et la dégradation de la topoisomérase IIα et la condensation/décondensation chromosomique (Heck *et al*., 1988).

Les connaissances actuelles concernant la régulation du gène de la topoisomérase IIα restent somme toute assez sommaires. Récemment, une région promotrice d'environ 650 paires de bases a été décrite par Hochhauser *et al*. (1992), elle présente toutes les caractéristiques d'un gène domestique, absence de boîte TATA et richesse modérée en sites GC (présence notamment d'une boîte Sp 1 pouvant remplacer la boîte TATA) en sont deux exemples. La présence de 5 boîtes CCAAT inversées ou ICB (Inverted CCAAT box) est une autre particularité de ce type de promoteur.

Des facteurs de transcription interagissant avec le promoteur du gène de la topoisomérase IIα humaine ont été décrits ; on peut citer c-myb (Brandt *et al*., 1997), p53 (Sandri *et al*., 1996), ATF (Lim *et al*., 1998), Sp1 et Sp3 (Kubo *et al*., 1995). Quoi qu'il en soit, en dehors de NF-Y (également appelé CBF, ACF et CP1, références dans Isaacs *et al*., 1996) les facteurs de transcription agissant sur les séquences ICB du promoteur du gène de la topoisomérase IIα humaine ne sont pas encore tous identifiés et caractérisés ; Herzog et Zwelling (1997) ont cependant mis en évidence deux protéines d'un poids moléculaire apparent de 90 kD et de 140 kD qui lient respectivement ICB1 à ICB4 et ICB5. Isaacs et ses collaborateurs (1996) ont proposé que le NFY ainsi qu'une autre protéine non identifiée reconnaissent une boîte ICB de la région promotrice du gène de la topoisomérase IIα ; ils ont également montré que les mutations de ICB2 abrogeaient complètement la diminution de l'activité promotrice normalement observée dans des cellules arrêtées à confluence (Isaac *et al*., 1996). Ils ont identifié NFY comme un composant d'un complexe induit par la prolifération et qui se lie *in vitro* à la séquence ICB2 du promoteur du gène de la topoisomérase IIα humaine, bien que NF-Y soit toujours détectable dans les cellules arrêtées à confluence (Isaac *et al*., 1996). Ils ont proposé que ICB2 agisse comme un régulateur négatif du promoteur du gène de la topoisomérase IIα des cellules arrêtées à confluence et que cette répression puisse être supprimée dans les cellules prolifératives. La boite ICB2 du promoteur du gène de la topoisomérase IIα joue donc un rôle primordial dans l'arrêt du processus prolifératif normal lorsque les cellules arrivent à confluence.

Des facteurs de transcription se liant à la séquence ICB ainsi que la séquence ICB elle-même constituent donc des cibles moléculaires pour contrôler le taux d'expression de la topoisomérase IIα. En intervenant sur ces facteurs, il est possible d'envisager de contrôler l'expression du gène de la topoisomérase IIα et par voie de conséquence la prolifération cellulaire.

La présente invention a pour objet la mise en évidence de nouveaux facteurs de transcription se liant la boite ICB impliquée dans le contrôle de la prolifération cellulaire.

Une technique récente appelée système « simple-hybride » qui permet d'isoler des clones ADNc codant pour des protéines de liaison à l'ADN spécifique de certaines séquences a été utilisée. Ce système présente un double avantage car il est capable non seulement de mettre à jour des interactions ADN-protéine *in vivo* chez la levure mais aussi de donner directement accès aux ADN complémentaires (ADNc) codant les protéines candidates ayant une activité de facteur transcription. Le système repose principalement sur la construction d'une souche de levure test selon le principe mis au point par Wang et Reed (1993). Cette souche de levure permet le criblage de banques d'ADNc en mettant en évidence l'interaction ADN-protéine *in vivo* par le biais de l'activation d'un gène rapporteur intégré au génome de la levure test.

La présente invention a donc pour objet un polypeptide isolé dénommé ICBP90 (inverted CCAAT box binding protein) de séquence d'acides aminés SEQ ID N°2. Cette séquence comprend :
a) un domaine « ubiquitine » comprenant la séquence d'acides aminés 1 à 75 de la séquence SEQ ID N°2 ;
b) un domaine « doigt de zinc » de type C4HC3 comprenant la séquence d'acides aminés 310 à 366 de la séquence SEQ ID N°2 et un domaine "doigt de zinc" de type C3HC4 comprenant la séquence d'acides aminés 724 à 763 de la séquence ID n° 2;
c) un domaine « leucine zipper » putatif comprenant la séquence d'acides aminés 58 à 80 de la séquence SEQ ID N°2 ;
d) deux domaines de localisation nucléaire potentiels comprenant les séquences d'acides aminés 581 à 600 et 648 à 670 de la séquence SEQ ID N°2 ;
e) un site de phosphorylation par une tyrosine kinase comprenant la séquence d'acides aminés 452 à 458 de la séquence SEQ ID N°2 ;
f) des sites de phosphorylation par une protéine kinase cAMP/cGMP dépendante comprenant les séquences d'acides aminés 246 à 249, 295 à 298 et 648 à 651 de la séquence SEQ ID N°2 ;
g) des sites de phosphorylation par une caséine kinase II comprenant la séquence d'acides aminés 23 à 26, 57 à 60, 91 à 94, 109 à 112, 165 à 168, 265 à 268, 354 à 357 et 669 à 672 de la séquence SEQ ID N°2 ;
h) des sites de phosphorylation par une protéine kinase C comprenant la séquence d'acides aminés 82 à 84, 104 à 106, 160 à 162, 173 à 175, 251 à 253, 301 à 303, 380 à 382, 393 à 395, 504 à 506, 529 à 531, 625 à 627 et 639 à 641 de la séquence SEQ ID N°2.

Il est à noter que Fujimori et al (Mammalian Genome, 9 :1032) décrit par ailleurs une protéine nucléaire murine appelée Np95 dont la séquence nucléotidique présente une identité de séquence de 77,6% avec la séquence nucléotidique SEQ ID NO1 de l'invention, et dont la séquence protéique présente une identité de séquence de 73,6% avec la SEQ ID NO2 de l'invention. Cependant, le profil d'expression de Np95 diffère largement de celui de la protéine ICBP90 de l'invention : à l'inverse de I'ICBP90, Np95 est fortement exprimée dans la rate, le poumon, et les testicules. Les fonctions biologiques de ces deux protéines paraissent donc clairement distinctes malgré l'homologie de séquence.

La présente invention porte également sur un polypeptide isolé caractérisé en ce qu'il comprend un polypeptide choisi parmi :
a) un polypeptide de séquence SEQ ID N°2, SEQ ID N°4, SEQ ID N°6, SEQ ID N°8; ou le fragment C terminal à partir du residu D263 de SEQ ID NO2

Il doit être compris que l'invention concerne les polypeptides obtenus par purification à partir de sources naturelles, ou bien obtenues par recombinaison génétique, ou encore par synthèse chimique et pouvant alors comporter des acides aminés non naturels.

Dans la présente description, on utilisera le terme polypeptide pour désigner également une protéine ou un peptide.

On entendra par polypeptide variant l'ensemble des polypeptides mutés pouvant exister naturellement, en particulier chez l'être humain, et qui correspondent notamment à des troncatures, substitutions, délétions et/ou additions de résidus d'amino-acides. Les polypeptides homologues selon l'invention conserve au moins un domaine choisi parmi le domaine de liaison à l'ADN et/ou le domaine d'interaction avec une autre protéine.

Par polypeptide homologue, on entendra désigner les polypeptides présentent, par rapport au polypeptide naturel ICBP90, certaines modifications comme en particulier une délétion, addition ou substitution d'au moins un acide aminé, une troncature, un allongement et/ou une fusion chimérique. Parmi les polypeptides homologues, on préfère ceux dont la séquence d'acides aminés présente au moins 80 % d'homologie, de préférence 90 %, de manière préférée 95 %, et de manière encore préférée 97 % d'homologie avec les séquences d'acides aminés des polypeptides selon l'invention. Dans le cas d'une substitution, un ou plusieurs acides aminés consécutifs ou non consécutifs, sont remplacés par des acides aminés « équivalents ». L'expression acide aminé « équivalent » vise ici à désigner tout acide aminé susceptible d'être substitué à l'un des acides aminés de la structure de base sans cependant modifier les caractéristiques ou propriétés fonctionnelles essentielles, comme leurs activités biologiques, des polypeptides correspondants telles que l'induction *in vivo* d'anticorps capables de reconnaître le polypeptide dont la séquence d'acides aminés est comprise dans la séquence d'acides aminés SEQ ID N°2, ou l'un de ses fragments ci-dessus définis et notamment la séquence d'acides aminés SEQ ID N°4, SEQ ID N°6 et SEQ ID N°8. Ces acides aminés équivalents peuvent être déterminés soit en s'appuyant sur leur homologie de structure avec les acides aminés auxquels ils se substituent, soit sur les résultats des essais d'activité biologique croisée auxquels les différents polypeptides sont susceptibles de donner lieu. A titre d'exemple, on mentionnera les possibilités de substitutions susceptibles d'être effectuées sans qu'il en résulte une modification approfondie des activités biologiques des polypeptides modifiés correspondants, les remplacements, par exemple, de la leucine par la valine ou l'isoleucine, de l'acide aspartique par l'acide glutamique, de la glutamine par l'asparagine, de l'arginine par la lysine etc., les substitutions inverses étant naturellement envisageables dans les mêmes conditions.

Par fragment biologiquement actif, on entendra désigner en particulier un fragment de séquence d'acides aminés de polypeptide selon l'invention présentant au moins une des caractéristiques ou propriétés fonctionnelles des polypeptides selon l'invention, notamment en ce que : (i) il est capable d'être reconnu par un anticorps spécifique d'un polypeptide selon l'invention ; (ii) il présente au moins l'un des domaines ou régions tels que définis ci-après ; (iii) il est capable de se lier à l'ADN et notamment aux boîtes CCAATT et/ou CCAAT inversée ; (iv) il est capable de moduler le taux d'expression du gène de la topoisomérase IIα ; (v) il est capable de moduler la prolifération cellulaire.

Par fragment de polypeptide, on entend désigner un polypeptide comportant au minimun 5 acides aminés, de préférence 7 acides aminés, de manière préférée 10 et de manière encore préférée 15 acides aminés. Les fragments de polypeptide selon l'invention obtenus par clivage dudit polypeptide par une enzyme protéolytique, par un réactif chimique, ou encore en plaçant ledit polypeptide dans un environnement très acide font également partie de l'invention.

Le polypeptide selon l'invention peut également s'associer à d'autres polypeptides par des interactions protéine-protéine. On entend désigner par interactions protéine-protéine, des associations mettant directement en contact au moins deux protéines. Ainsi, le polypeptide de l'invention peut se dimériser pour former des homodimères ou des hétérodimères, ou s'associer sous la forme d'homomultimères ou d'hétéromultimères. Le polypeptide selon l'invention peut également interagir avec un autre polypeptide pour exercer son action ; ainsi, le polypeptide selon l'invention peut posséder, en plus de son domaine de liaison à l'ADN, un domaine d'action sur la transcription qui exerce son action via des interactions protéine-protéine avec d'autres composants protéique de la machinerie transcriptionnelle. On entend désigner par composant protéique de la machinerie transcriptionnelle tous les facteurs de transcription nécessaires à la réalisation et à la régulation de la réaction de transcription.

Le polypeptide selon l'invention est caractérisé en ce qu'il est capable de se lier à une séquence d'ADN et en ce qu'il est comporte au moins un domaine de fixation à l'ADN sélectionné dans le groupe composé d'un domaine « doigt de zinc » (zinc-finger) et d'un domaine « leucine zipper » la séquence d'ADN sur laquelle se lie ledit polypeptide est une boite CCAAT, de préférence une une boite CCAAT inversée (inverted CCAAT box : ICB).

On entend désigner par liaison à une séquence d'ADN, une interaction spécifique entre le polypeptide de l'invention et une séquence d'ADN au moyen d'une série de liaisons faibles contractées entre les acides aminés de la protéine et les bases. Le polypeptide selon l'invention possède au moins un domaine de liaison à l'ADN qui contient au moins un des motifs protéiques connus susceptibles d'interagir avec l'ADN, c'est-à-dire la structure en doigt de gant à laquelle est associée un atome de zinc (« zinc-finger »), la structure hélice-tour-hélice, la structure hélice-boucle-hélice et la fermeture éclair à leucines (« leucine-zipper »).

Par motif en doigt de gant (« zinc-finger »), on entend désigner une séquence d'une vingtaine d'acides aminés ayant dans l'espace une forme de doigt de gant. Il en existe deux types : ceux qui contiennent quatre cystéines (C4) et ceux qui contiennent deux cystéines et deux histidines (C2H2). Ces acides aminés définissent la nature du doigt de gant et sont situés à sa base et un ion Zn++ est situé au centre du carré formé par ces quatre acides aminés. Le polypeptide selon l'invention possède potentiellement deux motifs de type C4.

Par motif de type « leucine zipper », on entend désigner des motifs appartenant à des facteurs de transcription dimérique qui sont soit des homodimères, soit des hétérodimères. Le monomère est constitué d'une séquence à caractère basique qui interagit de manière spécifique avec l'ADN et d'un domaine hydrophobe en hélice α qui interagit avec le domaine homologue de l'autre chaîne. Dans ce domaine se trouve une leucine tous les 7 aminoacides, c'est-à-dire à chaque tour d'hélice. Toutes ces leucines sont alignées et l'interaction se fait à leur niveau entre les deux monomères. Le polypeptide selon l'invention possède potentiellement un motif de type « leucine zipper ».

L'invention concerne également un polynucléotide isolé caractérisé en ce qui code pour un polypeptide de séquence SEQ ID N° 1 tel que défini précédemment. De manière préférée, le polynucléotide selon l'invention possède la séquence SEQ ID N°1.

L'invention concerne également le polynucléotide isolé caractérisé en ce qu'il comprend un polynucléotide choisi parmi :
a) un polynucléotide de séquence SEQ ID N°1, SEQ ID N°3, SEQ ID N°5 ou SEQ ID N°7 ou codant pour le fragment C terminal à partir du résidu D263 de SEQ ID NO2

Dans la présente description, on entendra désigner par polynucléotide, oligonucléotide, séquence de polynucléotide, séquence nucléotidique ou acide nucléique un fragment d'ADN, aussi bien un ADN double brin, un ADN simple brin que des produits de transcription desdits ADNs, et/ou un fragment d'ARN, lesdits fragments naturels isolés, ou de synthèse, comportant ou non des nucléotides non naturels, désignant un enchaînement précis de nucléotides, modifiés ou non, permettant de définir un fragment ou une région d'un acide nucléique.

Par polynucléotide de séquence complémentaire, on entend désigner tout ADN dont les nucléotides sont complémentaires de ceux de la SEQ ID N° 1, SEQ ID N°3, SEQ ID N°5, SEQ ID N°7 ou d'une partie de la SEQ ID N° 1, SEQ ID N°3, SEQ ID N°5, SEQ ID N°7 et dont l'orientation est inversée.

Par pourcentage d'homologie au sens de la présente invention, on entend un pourcentage d'identité entre les bases de deux polynucléotides, ce pourcentage étant purement statistique et les différences entre les deux polynucléotides étant réparties au hasard et sur toute leur longueur. Selon l'invention, les polynucléotides de séquence nucléique homologue présentent un taux d'homologie d'au moins 80 %, de préférence 90 %, de manière préférée 95 %, de manière encore préférée 97 %.

Une hybridation dans des conditions de forte stringence signifie que les conditions de température et de force ionique sont choisies de telle manière qu'elles permettent le maintien de l'hybridation entre deux fragments d'ADN complémentaires. A titre illustratif, des conditions de forte stringence de l'étape d'hybridation aux fins de définir les fragments polynucléotidiques décrits ci-dessus, sont avantageusement les suivantes :

L'hybridation ADN-ADN ou ADN-ARN est réalisée en deux étapes: (1) préhybridation à 42°C pendant 3 heures en tampon phosphate (20 mM, pH 7,5) contenant 5 x SSC ( 1 x SSC correspond à une solution 0,15 M NaCl + 0,015 M citrate de sodium), 50 % de formamide, 7 % de sodium dodécyl sulfate (SDS), 10 x Denhard's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille > 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-avant pour un polynucléotide de taille définie, seront adaptées par l'homme du métier pour des oligonucléotides de taille plus grande ou plus petite, selon l'enseignement de Sambrook *et al*., 1989.

Avantageusement, un fragment nucléotidique répondant à la définition précédente aura au moins 15 nucléotides consécutifs, de préférence au moins 21 nucléotides, et encore plus préférentiellement au moins 30 nucléotides consécutifs de la séquence dont il est issu.

Par EST (« expressed sequence tag »), on entend désigner des séquences exprimées, caractérisées dans une banque d'ADN complémentaire (ADNc) et utilisées comme balise cartographique de l'ADN génomique.

Un tel polynucléotide se caractérise en ce qu'il est marqué directement ou indirectement par un composé radioactif ou un composé non radioactif. Il peut être utilisé en tant qu'amorce pour l'amplification ou la polymérisation de séquences nucléiques ; ou en tant que sonde pour la détection de séquences nucléiques. Les fragments de polynucléotides pouvant être utilisés comme sonde ou comme amorce dans des procédés de détection, d'identification, de dosage ou d'amplification de séquence nucléique, présenteront une taille minimale de 9 bases, de préférence de 18 bases, et de manière plus préférée 36 bases. Enfin, il est possible d'utiliser un polynucléotide selon l'invention en tant que séquence d'acide nucléique sens ou antisens pour contrôler l'expression du produit protéique correspondant.

Les séquences de polynucléotides selon l'invention non marquées peuvent être utilisées directement comme sonde, amorce ou oligonucléotide ; cependant les séquences utilisées sont généralement marquées pour obtenir des séquences utilisables pour de nombreuses applications. Le marquage des amorces, des sondes, des oligonucléotides selon l'invention est réalisé par des éléments radioactifs ou par des molécules non radioactives ; parmi les isotopes radioactifs utilisés, on peut citer le ³²P, le ³³P, le ³⁵S, le 3H ou le ¹²⁵I. Les entités non radioactives sont sélectionnées parmi les ligands tels la biotine, l'avidine, la streptavidine, la dioxygénine, les haptènes, les colorants, les agents luminescents tels que les agents radioluminescents, chémiluminescents, bioluminescents, fluorescents, phosphorescents.

Les polynucléotides selon l'invention peuvent ainsi être utilisés comme amorce et/ou sonde dans des procédés mettant en oeuvre notamment la technique PCR (réaction en chaîne à la polymérase)(Erlich, 1989 ; Innis *et al*., 1990, et Rolfs *et al*., 1991). Cette technique nécessite le choix de paires d'amorces oligonucléotidiques encadrant le fragment qui doit être amplifié. On peut, par exemple, se référer à la technique décrite dans le brevet américain U.S. N° 4 683 202. Les fragments amplifiés peuvent être identifiés, par exemple après une électrophorèse en gel d'agarose ou de polyacrylamide, ou après une technique chromatographique comme la filtration sur gel ou la chromatographie échangeuse d'ions. La spécificité de l'amplification peut être contrôlée par hybridation moléculaire en utilisant comme sonde les séquences nucléotidiques de polynucléotides de l'invention, des plasmides contenant ces séquences ou leurs produits d'amplification. Les fragments nucléotidiques amplifiés peuvent être utilisés comme réactifs dans des réactions d'hybridation afin de mettre en évidence la présence, dans un échantillon biologique, d'un acide nucléique cible de séquence complémentaire à celle desdits fragments nucléotidiques amplifiés.

L'invention vise également les fragments nucléotidiques susceptibles d'être obtenus par amplification à l'aide d'amorces selon l'invention.

D'autres techniques d'amplification de l'acide nucléique cible peuvent être avantageusement employées comme alternative à la PCR (PCR-like) à l'aide de couple d'amorces de séquences nucléotidiques selon l'invention. Par PCR-like on entendra désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues, en général il s'agit de l'amplification de l'ADN par une polymérase ; lorsque l'échantillon d'origine est un ARN il convient préalablement d'effectuer une transcription inverse. Il existe actuellement de très nombreux procédés permettant cette amplification, comme par exemple la technique SDA (Strand Displacement Amplification) ou technique d'amplification à déplacement de brin (Walker *et al*., 1992), la technique TAS (Transcription-based Amplification System) décrite par Kwoh *et al*. en 1989, la technique 3SR (Self-Sustained Sequence Replication) décrite par Guatelli *et al*. en 1990, la technique NASBA (Nucleic Acid Sequence Based Amplification) décrite par Kievitis *et al*. en 1991, la technique TMA (Transcription Mediated Amplification), la technique LCR (Ligase Chain Reaction) décrite par Landegren *et al*. en 1988 et perfectionnée par Barany *et al*. en 1991, qui emploie une ligase thermostable, la technique de RCR (Repair Chain Reaction) décrite par Segev en 1992, la technique CPR (Cycling Probe Reaction) décrite par Duck *et al*. en 1990, la technique d'amplification à la Q-béta-réplicase décrite par Miele *et al*. en 1983 et perfectionnée notamment par Chu *et al*. en 1986 et Lizardi *et al*. en 1988, puis par Burg *et al*. ainsi que par Stone *et al*. en 1996.

Dans le cas où le polynucléotide cible à détecter est un ARN, par exemple un ARNm, on utilisera avantageusement, préalablement à la mise en oeuvre d'une réaction d'amplification à l'aide des amorces selon l'invention ou à la mise en oeuvre d'un procédé de détection à l'aide des sondes de l'invention, une enzyme de type transcriptase inverse afin d'obtenir un ADNc à partir de l'ARN contenu dans l'échantillon biologique. L'ADNc obtenu servira alors de cible pour les amorces ou les sondes mises en oeuvre dans le procédé d'amplification ou de détection selon l'invention.

Les sondes nucléotidiques hybrident spécifiquement avec une molécule d'ADN ou d'ARN de polynucléotide, plus particulièrement avec la séquence SEQ ID N° 1 codant pour le polypeptide ICBP90, dans des conditions d'hybridation de forte stringence telles que données sous forme d'exemple précédemment.

La technique d'hybridation peut être réalisée de manières diverses (Matthews *et al*., 1988). La méthode la plus générale consiste à immobiliser l'acide nucléique extrait des cellules de différents tissus ou de cellules en culture sur un support (tel que la nitrocellulose, le nylon, le polystyrène) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde).

Selon un autre mode de mise en oeuvre des sondes nucléiques, ces dernières peuvent être utilisées comme sonde de capture. Dans ce cas, une sonde, dite « sonde de capture », est immobilisée sur un support et sert à capturer par hybridation spécifique l'acide nucléique cible obtenu à partir de l'échantillon biologique à tester et l'acide nucléique cible est ensuite détecté grâce à une seconde sonde, dite « sonde de détection », marquée par un élément facilement détectable.

L'invention concerne un vecteur recombinant de clonage d'un polynucléotide selon l'invention et/ou d'expression d'un polypeptide selon l'invention caractérisé en ce qu'il contient un polynucléotide selon l'invention tel que précédemment décrit. Le vecteur selon l'invention est caractérisé en ce qu'il comporte les éléments permettant l'expression éventuellement la sécrétion desdites séquences dans une cellule hôte. Ces vecteurs sont utiles pour transformer des cellules hôtes afin de cloner ou d'exprimer les séquences nucléotidiques de l'invention. Des vecteurs particuliers sont par exemple des vecteurs d'origine virale ou plasmidique. Parmi ces vecteurs, on préfère ceux de la série pGEX (Pharmacia) pour l'expression dans les bactéries ou pSG5 (Stratagene, La Jolla, CA USA) pour l'expression en système eucaryote.

Selon un mode particulier de réalisation, le vecteur selon l'invention comporte des éléments de contrôle de l'expression des polypeptides ; ces éléments de contrôle sont choisis de préférence parmi (i) la séquence promotrice du gène ICBP90 selon l'invention qui correspond à la séquence SEQ ID N°12 ; (ii) un polynucléotide dont la séquence est complémentaire à la séquence SEQ ID N° 12 ; (iii) un polynucléotide dont la séquence comporte au moins 80% d'identité avec un polynucléotide défini en (i) ou (ii) ; (iv) un polynucléotide hybridant dans des conditions de forte stringence avec la séquence de polynucléotide définie en (i), (ii), (iii). Les outils informatiques à la disposition de l'homme du métier lui permettent aisément d'identifier les boites régulatrices promotrices nécessaires et suffisantes au contrôle de l'expression génique, notamment les boites TATA, CCAAT, GC, ainsi que les séquences régulatrices stimulatrices (« enhancer ») ou inhibitrices (« silencers ») qui contrôlent en CIS l'expression des gènes selon l'invention.

L'invention comprend en outre les cellules hôtes, notamment les cellules eucaryotes et procaryotes, caractérisées en ce qu'elles comprennent les vecteurs selon l'invention. De préférence, les cellules hôtes sont transformées dans des conditions permettant l'expression d'un polypeptide recombinant selon l'invention. L'hôte cellulaire peut être choisi parmi les cellules bactériennes (Olins et Lee, 1993), mais également les cellules de levure (Buckholz, 1993), de même que les cellules animales, en particulier les cultures de cellules de mammifères (Edwards et Aruffo, 1993), mais également les cellules d'insectes dans lesquelles on peut utiliser des procédés mettant en oeuvre des baculovirus par exemple (Luckow, 1993). Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

L'invention concerne également une méthode de préparation d'un polypeptide caractérisé en ce qu'il met en oeuvre un vecteur selon l'invention. Plus particulièrement, l'invention porte sur une méthode de préparation d'un polypeptide recombinant caractérisé en ce que l'on cultive des cellules transformées selon l'invention dans des conditions permettant l'expression dudit polypeptide recombinant et que l'on récupère ledit polypeptide recombinant.

Le polypeptide selon l'invention est susceptible d'être obtenu selon un procédé de l'invention et selon les techniques de production de polypeptides recombinants connues de l'homme du métier. La présente invention concerne donc le polypeptide recombinant susceptible d'être obtenu par la méthode ci-dessus présentée. Dans ce cas, la séquence d'acide nucléique utilisée est placée sous le contrôle de signaux permettant son expression dans un hôte cellulaire. Un système efficace de production d'un polypeptide recombinant nécessite de disposer d'un vecteur, par exemple d'origine plasmidique ou virale, et d'une cellule hôte compatible. Le vecteur doit comporter un promoteur, des signaux d'initiation et de terminaison de la traduction, ainsi que des régions appropriées de régulation de la transcription. Il doit pouvoir être maintenu de façon stable dans la cellule et peut éventuellement posséder des signaux particuliers spécifiant la sécrétion du polypeptide traduit. Ces différents signaux de contrôle sont choisis en fonction de l'hôte cellulaire utilisé. A cet effet, les séquences d'acide nucléique selon l'invention peuvent être insérées dans des vecteurs à réplication autonome au sein de l'hôte choisi, ou des vecteurs intégratifs de l'hôte choisi. De tels vecteurs seront préparés selon les méthodes couramment utilisées par l'homme du métier, et les clones en résultant peuvent être introduits dans un hôte approprié par des méthodes standards telles par exemple la transfection par précipitation au phosphate de calcium, la lipofection, l'électroporation, le choc thermique.

Les polypeptides recombinants obtenus comme indiqué ci-dessus, peuvent aussi bien se présenter sous forme glycosylée que non glycosylée et peuvent présenter ou non la structure tertiaire naturelle.

Les polypeptides obtenus par synthèse chimique et pouvant comporter des acides aminés non naturels correspondant auxdits polypeptides recombinants, sont également compris dans l'invention. Les peptides selon l'invention peuvent également être préparés par les techniques classiques, dans le domaine de la synthèse des peptides. Cette synthèse peut être réalisée en solution homogène ou en phase solide.

Les procédés de purification de polypeptide recombinant utilisés sont connus de l'homme du métier. Le polypeptide recombinant peut être purifié à partir de lysats et extraits cellulaires, du surnageant du milieu de culture, par des méthodes utilisées individuellement ou en combinaison, telles que le fractionnement, les méthodes de chromatographie, les techniques d'immuno-affinité à l'aide d'anticorps mono ou polyclonaux spécifiques, etc.

Une variante préférée consiste à produire un polypeptide recombinant fusionné à une protéine « porteuse » (protéine chimère). L'avantage de ce système est qu'il permet une stabilisation et une diminution de la protéolyse du produit recombinant, une augmentation de la solubilité au cours de la renaturation *in vitro* et/ou une simplification de la purification lorsque le partenaire de fusion possède une affinité pour un ligand spécifique.

L'invention concerne également un anticorps monoclonal ou polyclonal et ses fragments, caractérisés en ce qu'ils lient spécifiquement un polypeptide choisi parmi SEQ ID NO: 2, 4 ou 8. Les anticorps chimériques, les anticorps humanisés et les anticorps simple chaîne font également partie de l'invention. Les fragments d'anticorps selon l'invention sont de préférence des fragments Fab ou F(ab')2.

Les polypeptides selon l'invention permettent de préparer des anticorps monoclonaux ou polyclonaux. Les anticorps monoclonaux pourront avantageusement être préparés à partir d'hybridomes selon la technique décrite par Kohler et Milstein en 1975. Les inventeurs ont employé cette technique pour obtenir un hybridome produisant un nouvel anticorps monoclonal hautement spécifique d'un épitope de la protéine ICBP90.

Les anticorps polyclonaux pourront être préparés, par exemple par immunisation d'un animal, en particulier une souris, avec un polypeptide selon l'invention associé à un adjuvant de la réponse immunitaire, puis purification des anticorps spécifiques contenus dans le sérum des animaux immunisés sur une colonne d'affinité sur laquelle a préalablement été fixé le polypeptide ayant servi d'antigène. Les anticorps polyclonaux selon l'invention peuvent aussi être préparés par purification sur une colonne d'affinité, sur laquelle a préalablement été immobilisé un polypeptide selon l'invention.

On peut également citer anticorps monoclonal spécifique de la protéine ICBP90 humaine et capable, d'inhiber l'interaction entre ICBP90 et la séquence d'ADN sur laquelle se lie spécifiquement la protéine ICBP90. Selon un autre mode de réalisation, l'anticorps monoclonal et spécifique de la protéine ICBP90 humaine est capable d'inhiber l'interaction entre ICBP90 et les protéines avec lesquelles ICBP90 interagit, lesdites protéines étant de préférence ICBP90 elle-même ou des protéines du complexe transcriptionnel. Par protéines du complexe transcriptionnel, on entend désigner toutes les protéines intervenant dans la réaction de la transcription que se soit l'initiation, l'élongation ou la terminaison de la transcription.

Les anticorps de l'invention pourront également être marqués de la même manière que décrit précédemment pour les sondes nucléiques de l'invention et de manière préférée avec un marquage de type enzymatique, fluorescent ou radioactif.

Par ailleurs, outre leur utilisation pour la purification des polypeptides, les anticorps de l'invention, en particulier les anticorps monoclonaux, peuvent également être utilisés pour la détection de ces polypeptides dans un échantillon biologique.

Ils constituent ainsi un moyen d'analyse de l'expression de polypeptide selon l'invention, par exemple par immunofluorescence, marquage à l'or, immunoconjugués enzymatiques.

Plus généralement, les anticorps de l'invention peuvent être avantageusement mis en oeuvre dans toute situation où l'expression d'un polypeptide selon l'invention doit être observée, et plus particulièrement en immunocytochimie, en immunohistochimie ou dans des expériences de « western blotting ».

Ainsi, l'invention concerne une méthode de détection et/ou de dosage d'un polypeptide selon l'invention, dans un échantillon biologique, caractérisé en ce qu'il comprend les étapes suivantes de mise en contact de l'échantillon biologique avec des anticorps selon l'invention puis de mise en évidence du complexe antigène-anticorps formé. Cette méthode peut être utilisée en immunocytochimie pour la localisation cellulaire du polypeptide selon l'invention et en immunohistochimie pour évaluer la prolifération cellulaire.

Entre également dans le cadre de l'invention, un nécessaire pour la détection et/ou le dosage d'un polypeptide selon l'invention dans un échantillon biologique, caractérisé en ce qu'il comprend les éléments suivants : (i) un anticorps monoclonal ou polyclonal tel que décrit précédemment ; (ii) le cas échéant, les réactifs pour la constitution du milieu propice à la réaction immunologique ; (iii) les réactifs permettant la détection des complexes antigène-anticorps produits par la réaction immunologique. Ce kit est notamment utile à la réalisation d'expériences de Western Blotting ; celles-ci permettent d'étudier la régulation de l'expression du polypeptide selon l'invention à partir de tissus ou de cellules. Ce kit est également utile aux expériences d'immunoprécipitation pour mettre en évidence notamment les protéines interagissant avec le polypeptide selon l'invention.

Toute procédure classique peut être mise en oeuvre pour réaliser une telle détection et/ou dosage. A titre d'exemple, une méthode préférée met en jeu des processus immunoenzymatiques selon la technique ELISA, par immunofluorescence, ou radio-immunologique (RIA) ou équivalent.

On peut également citer une méthode de détection et/ou de dosage d'acide nucléique, dans un échantillon biologique, caractérisé en ce qu'il comporte les étapes suivantes : (i) d'isolement de l'ADN à partir de l'échantillon biologique à analyser, ou obtention d'un ADNc à partir de l'ARN de l'échantillon biologique ; (ii) d'amplification spécifique de l'ADN codant pour le polypeptide selon l'invention à l'aide d'amorces ; (iii) d'analyse des produits d'amplification.

Un nécessaire utile pour la détection et/ou le dosage d'un acide nucléique, dans un échantillon biologique ci-dessus mentionné, comprend les éléments suivants : (i) un couple d'amorces nucléiques selon l'invention, (ii) les réactifs nécessaires pour effectuer une réaction d'amplification d'ADN, et éventuellement (üi) un composant permettant de vérifier la séquence du fragment amplifié, plus particulièrement une sonde selon l'invention.

Une autre méthode de détection et/ou de dosage d'acide nucléique, dans un échantillon biologique, comporte les étapes suivantes : (i) de mise en contact d'une sonde selon l'invention avec un échantillon biologique ; (ii) de détection et/ou de dosage de l'hybride formé entre ladite sonde et l'ADN de l'échantillon biologique.

Un nécessaire utile pour cette détection et/ou ce dosage d'acide nucléique, dans un échantillon biologique, comprend les éléments suivants : (i) une sonde selon l'invention, (ii) les réactifs nécessaires à la mise en oeuvre d'une réaction d'hybridation, et le cas échéant, (iii) un couple d'amorces selon l'invention, ainsi que les réactifs nécessaires à une réaction d'amplification de l'ADN.

L'invention concerne particulièrement les procédés selon l'invention et décrits ci-dessus, pour la détection et le diagnostic de prolifération cellulaire, et plus particulièrement de prolifération cellulaire d'origine cancéreuse.

L'invention concerné également une méthode de criblage de ligands susceptibles d'affecter l'activité transcriptionnelle d'un gène dont le promoteur comporte des boites CCAAT et/ou CCAAT inversées susceptibles de lier un polypeptide selon l'invention, ladite méthode étant caractérisée en ce qu'elle comporte les étapes suivantes de mise en contact dudit polypeptide et d'un ou plusieurs ligand(s) potentiel(s) en présence de réactifs nécessaires à la mise en oeuvre d'une réaction de transcription ou de détection et/ou de mesure de l'activité transcriptionnelle. C'est également un des objets de l'invention de fournir un kit ou un nécessaire pour le criblage de ligands susceptibles d'affecter l'activité transcriptionnelle d'un gène dont le promoteur comporte des boites CCAAT et/ou CCAAT inversées susceptibles de lier un polypeptide selon l'invention caractérisé en ce qu'il comprend les éléments suivants : (i) un polypeptide selon l'invention ; (ii) un ligand ; (iii) les réactifs nécessaires à la mise en oeuvre d'une réaction de transcription.

Le polypeptide ICBP90 selon l'invention présente une fonction de récepteur nucléaire. Par récepteur nucléaire, on entend désigner un polypeptide qui possède les propriétés essentielles des récepteurs nucléaires d'hormones. Cette superfamille de gène contient entre autres les récepteurs nucléaires à l'acide rétinoïque (RAR, RXR,...), les récepteurs nucléaires aux hormones stéroïdes (glucocorticoïdes, minéralocorticoïdes, progestérone, androgène, oestrogène), et les récepteurs nucléaires aux hormones thyroïdiennes (hormone T3). C'est donc également un des objets de la présente invention de fournir un procédé de criblage de ligand susceptible d'affecter la fonction « récepteur nucléaire » du polypeptide selon l'invention. Un tel procédé comporte les étaptes de:
a) mise en contact du polypeptide de l'invention et d'un ou plusieurs ligands potentiels en présence de réactifs nécessaires ;
b) détection et/ou mesure de l'activité transcriptionnelle d'un gène dont le promoteur comporte des séquences nucléotidiques sur lesquelles sont susceptibles de se lier le polypeptide de l'invention. De préférence, lesdites séquences nucléotidiques sont des boîtes CCAAT et/ou CCAAT inversées (ICB).

Les techniques de détection et/ou de mesure de l'activité transcriptionnelle sont connues de l'homme du métier. Il convient notamment de citer les technologies de Northern Blotting et de RT-PCR qui peuvent être mises en oeuvre avec les polynucléotides de l'invention utilisés respectivement comme sonde ou comme amorce.

Par ligand, on entend définir tous les composés susceptibles d'interagir avec le polypeptide selon l'invention pour former un complexe susceptible d'affecter l'activité transcriptionnelle, c'est-à-dire d'augmenter, de diminuer, de moduler ou d'annuler la transcription d'un gène sous le contrôle d'un promoteur contenant une séquence d'ADN à laquelle se lie le polypeptide de l'invention.

Un tel ligand est donc susceptible d'avoir une activité agoniste ou antagoniste. Parmi les ligands selon l'invention, il convient de citer les molécules biologiques interagissant avec le polypeptide selon l'invention, ainsi que tous les composés chimiques de synthèse. Parmi les ligands, il convient également de citer l'anticorps selon l'invention, ainsi qu'un oligonucléotide présentant une identité de séquence avec la séquence nucléotidique CCAAT et/ou CCAAT inversée ; un tel ligand est susceptible de constituer un inhibiteur du polypeptide selon l'invention.

L'invention porte également sur le ligand susceptible d'être obtenu par les procédés de criblage précédents.

On entend définir également par ligand tout composé susceptible de se lier à la séquence d'ADN de liaison du polypeptide selon l'invention. Un tel ligand constitue un inhibiteur compétitif du polypeptide selon l'invention pour la liaison à la séquence d'ADN.

De préférence, l'échantillon biologique selon l'invention dans lequel est réalisé la détection et le dosage est constitué par un fluide corporel, par exemple un sérum humain ou animal, du sang, de la salive, du mucus pulmonaire, ou par des biopsies. Entre également dans la définition d'un échantillon biologique de l'invention le liquide biologique résultant d'un lavage bronchoalvéolaire obtenu également lors des analyses diagnostiques des cancers des voies aériennes profondes.

Selon un autre aspect, l'invention concerne un composé caractérisé en ce qu'il est choisi parmi un anticorps, un polypeptide, un ligand, un polynucléotide, un oligonucléotide ou un vecteur selon l'invention à titre de médicament et notamment en tant que principes actifs de médicament ; ces composés seront préférentiellement sous forme soluble, associés à un véhicule pharmaceutiquement acceptable. Par véhicule pharmaceutiquement acceptable, on entend désigner tout type de véhicule employé habituellement dans la préparation de compositions injectables, c'est-à-dire un diluant, un agent de suspension tel une solution saline isotonique ou tamponnée. De préférence, ces composés seront administrés par voie systémique, en particulier par voie intraveineuse, par voie intramusculaire, intradermique ou par voie orale. Leurs modes d'administration, posologies et formes galéniques optimaux peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés, etc.

Selon un autre aspect, l'invention concerne un composé caractérisé en ce qu'il est choisi parmi un polypeptide, un polynucléotide, un polynucléotide antisens, un anticorps, un vecteur, une cellule, un ligand selon l'invention à titre de médicament et notamment en tant que principes actifs de médicament ; ces composés seront préférentiellement sous forme soluble, associés à un véhicule pharmaceutiquement acceptable. Par véhicule pharmaceutiquement acceptable, on entend désigner tout type de véhicule employé habituellement dans la préparation de compositions injectables, c'est-à-dire un diluant, un agent de suspension tel une solution saline isotonique ou tamponnée. De préférence, ces composés seront administrés par voie systémique, en particulier par voie intraveineuse, par voie intramusculaire, intradermique ou par voie orale. Leurs modes d'administration, posologies et formes galéniques optimaux peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés, etc. Quand l'agent est un polypeptide, un antagoniste, un ligand, un polynucléotide, par exemple une composition anti-sens, un vecteur, on peut l'introduire dans des tissus ou des cellules hôtes par un certain nombre de façons, incluant l'infection virale, la micro-injection ou la fusion de vésicules. On peut également utiliser l'injection par jet pour une administration intramusculaire comme décrit par Furth *et al*. (1992). On peut déposer le polynucléotide sur des microparticules d'or, et le délivrer par voie intradermique à l'aide d'un dispositif de bombardement de particules, ou un « pistolet à gène » comme décrit dans la littérature (voir par exemple Tang *et al.* (1992) où les microprojectiles d'or sont revêtues avec le polynucléotide de l'invention, de préférence le polynucléotide antisens de l'invention, puis bombardée dans les cellules de peau.

Le composé selon l'invention est utilisé pour la préparation d'un médicament destiné à moduler, à augmenter ou à diminuer la prolifération cellulaire.

L'invention porte également sur une composition pharmaceutique pour le traitement préventif et curatif du cancer caractérisée en ce qu'elle contient une quantité thérapeutiquement efficace d'un composé selon l'invention et un véhicule pharmaceutiquement acceptable. Selon un mode préféré de réalisation, la composition pharmaceutique est caractérisée en ce qu'elle contient un anticorps selon l'invention en tant qu'agent de ciblage conjugué à au moins un agent sélectionné parmi le groupe des agents antiprolifératifs, antinéoplastiques ou cytotoxiques. Ces agents sont des radioisotopes ou des entités non isotopiques. La conjugaison de l'anticorps de la présente invention à un agent antiprolifératif, antinéoplastique ou cytotoxique peut être utilisé pour arrêter le développement des cancers et pour induire une régression et/ou une élimination de la masse tumorale. De préférence, l'anticorps ou le fragment d'anticorps ainsi conjugué est introduit dans le patient atteint de cancer et délivré aux sites tumoraux par voie orale ou parentérale dans un liquide transporteur pharmaceutiquement acceptable tel qu'une solution de sel physiologique. Alternativement, une solution ou une suspension d'anticorps ou de fragment d'anticorps conjugué à un agent peut être perfusée directement dans le tissu épithélial malin, cette méthode étant utilisée de préférence dans le cas où le cancer n'est pas métastasé.

Les radioisotopes préférés conjugués aux anticorps monoclonaux employés pour la thérapie sont des radioisotopes émetteurs de rayons gamma et de préférence l'Iode¹³¹, l'Yttrium⁹⁰, l'Or¹⁹⁹, le Palladium¹⁰⁰, le Cuivre⁶⁷, le Bismuth²¹⁷ et l'Antimoine²¹¹. Les radioisotopes émetteurs de rayons beta et alpha peuvent également être utilisés pour la thérapie. Les entités non isotopiques conjuguées aux anticorps monoclonaux employés pour la thérapie sont multiples et variés; on peut citer: (i) les antimétabolites telles les agents anti-folate, le méthotrexate, (ii) les analogues des purines et des pyrimidines (mercaptopurine, fluorouracile, 5-azacytidine), (iii) les antibiotiques, (iv) les lectines (ricine, abrine) et (iv) les toxines bactériennes (toxine diphtérique).

L'anticorps selon invention peut également être utilisé en tant qu'agent de ciblage pour cibler des cellules cytotoxiques telles les cellules T humaines, les monocytes ou les cellules NK sur le lieu de la tumeur métastasée ou non. Les cellules cytotoxiques peuvent être attachées à l'anticorps via le récepteur Fc situé à la surface de ces cellules ou via un anticorps intermédiaire présentant une double spécificité par exemple; de tels anticorps bispécifiques pour le ciblage des cellules cancéreuses peuvent être produits en fusionnant une cellule immunitaire produisant l'anticorps de la présente invention ou l'hydridome de la présente invention avec une cellule produisant un anticorps dirigé contre la cellule cytotoxique à cibler. Des anticorps bispécifiques peuvent également être produits par couplage chimique de deux anticorps ayant la spécificité désirée. L'anticorps selon l'invention permet également de cibler des véhicules de délivrance d'agents antiprolifératifs, antinéoplastiques ou cytotoxiques sur le lieu de la tumeur métastasée ou non. Par véhicules de délivrance on entend désigner les liposomes et les particules virales. Dans certains cas, on pourra prévoir des éléments de ciblage assurant une expression spécifique de certains tissus ou cellules de façon à pouvoir limiter les zones d'expression des polypeptides selon l'invention.

L'invention concerne également un produit comprenant au moins un composé selon l'invention et au moins un agent anticancéreux comme produit de combinaison pour une utilisation simultanée, séparée ou étalée dans le temps en thérapie anti-cancéreuse.

Enfin, l'invention concerne une composition pour la détection, la localisation et l'imagerie des cancers, comprenant un anticorps selon l'invention, tel que l'anticorps est marqué directement ou indirectement avec un marqueur générateur de signal sélectionné parmi les isotopes radioactifs et les entités non isotopiques tels que définis précédemment. L'invention a également pour objet une méthode de détection, de localisation et d'imagerie du cancer, comprenant (i) les étapes d'injection parentérale chez un être humain d'une composition selon l'invention; (ii) l'accumulation après un temps suffisant au niveau des cellules cancéreuses de l'anticorps marqué puis pénétration de l'anticorps marqué à l'interieur desdites cellules, sans que ledit anticorps ne se lie de manière substantielle aux cellules normales; et (iii) la détection du signal au moyen d'un détecteur de signal; et (iv) la conversion du signal détecté en une image des cellules cancéreuses.

D'autres caractéristiques et avantages de l'invention apparaissent dans la suite de la description avec les exemples représentés ci-après. Dans ces exemples on se référera aux figures suivantes.

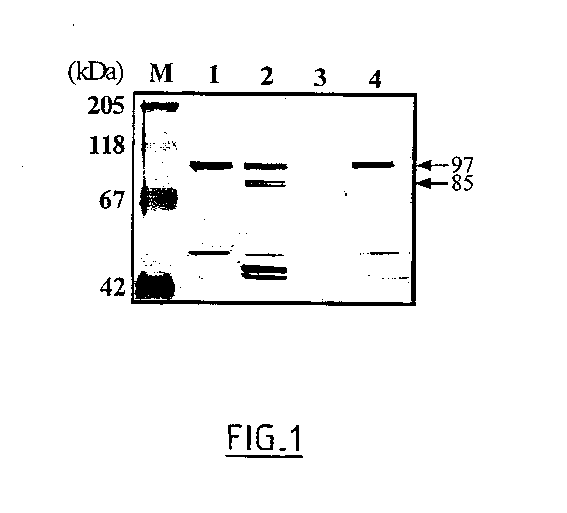

### Figure 1 : Expression de la protéine ICBP90 dans les cellules HeLa (cellules tumorales) et dans les fibroblastes pulmonaires humains en culture primaire (cellules non tumorales).

La détection de la protéine endogène ICBP90 a été réalisée sur des extraits totaux de protéines de cellules HeLa à confluence (piste 1) ou en prolifération (piste 2) et sur des extraits totaux de protéines de fibroblastes pulmonaires humains en culture primaire à confluence (piste 3) ou en prolifération (piste 4). Après migration sur gel de polyacrylamide 8% en présence de SDS, les protéines sont transférées sur membrane de nitrocellulose par électrotransfert. La révélation est réalisée à l'aide de l'anticorps 1RC1C-10 dilué au 1/4000 (concentration initiale 2 mg/ml) et d'un anticorps secondaire couplé à la phosphatase alcaline et dirigé contre les chaines lourdes d'anticorps de souris. Dans les pistes correspondant aux extraits de cellules HeLa, on observe une bande majeure à 97 kDa ; pour les cellules HeLa en prolifération, des bandes supplémentaires de tailles inférieures à 97 kDa apparaissent (piste 2). Dans les fibroblastes pulmonaires humains à confluence, la protéine endogène n'est pas exprimée (piste 3) et apparaît lorsque les cellules se mettent à proliférer (piste 4). Ces observations suggèrent que la protéine endogène ICBP90 est un marqueur de prolifération cellulaire pour des cellules normales (fibroblastes) tandis que pour les cellules tumorales elle serait un marqueur quelque soit le stade cellulaire.

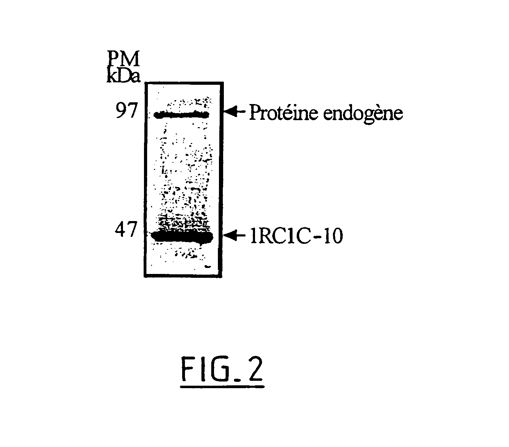

### Figure 2 : Immunoprécipitation de la protéine endogène

L'immunoprécipitation est réalisée sur des extraits protéiques totaux de cellules MOLT-4. L'anticorps 1RC1C-10 est fixé sur des billes de protéine G sépharose, puis mis en contact avec les-extraits protéiques pendant 2 heures à température ambiante. Après lavage les complexes billes/1RC1C-10/protéine sont précipités par centrifugation et analysés par migration sur gel de polyacrymalide 8% en présence de SDS, puis transfert sur membrane de nitrocellulose et révélation comme indiqué dans la figure 1. On observe une bande unique de 97 kDa, ainsi qu'une bande de 45 kDa qui correspond à la chaine lourde de 1RC1C-10.

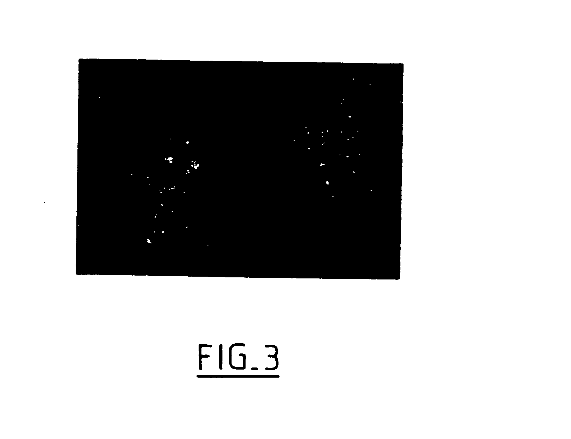

### Figure 3 : Localisation nucléaire de la protéine endogène

Nous avons utilisé des cellules HeLa pour examiner l'expression endogène de la protéine ICBP90 in situ à l'aide de l'anticorps 1RC1C-10 et d'un anticorps secondaire antisouris couplé au fluorochrome CY3. Le marquage est localisé exclusivement dans le noyau. Le nucléole et le cytoplasme ne sont pas marqués.

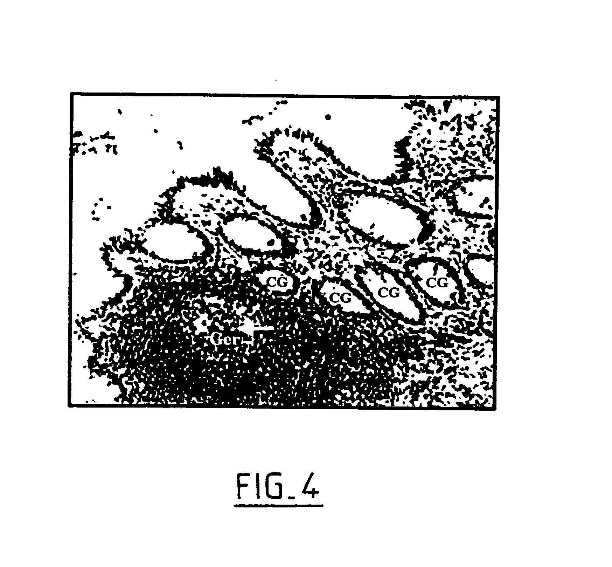

### Figure 4 : Expression de l'ICBP59 endogène dans les cellules en prolifération

Nous avons observé la protéine endogène sur des coupes en paraffine d'appendice humain. Après déparaffinage et prétraitement par chauffage en tampon acide (démasquage des sites antigéniques), les coupes sont incubées pendant 16 heures avec l'anticorps 1RC1C-10 dilué au 1/10000 (concentration initiale de 2 mg/ml). La révélation se fait par mise en contact avec un anticorps secondaire biotinylé, puis incubation avec le complexe streptavidine-péroxydase. Une contre coloration des noyaux à l'hématoxyline de Harris est également réalisée. Le marquage par 1RC1C-10 est localisé essentiellement dans des zones de prolifération cellulaire. Les cellules marquées se trouvent dans les cryptes glandulaires (CG) ainsi que dans les zones germinatives (ger).

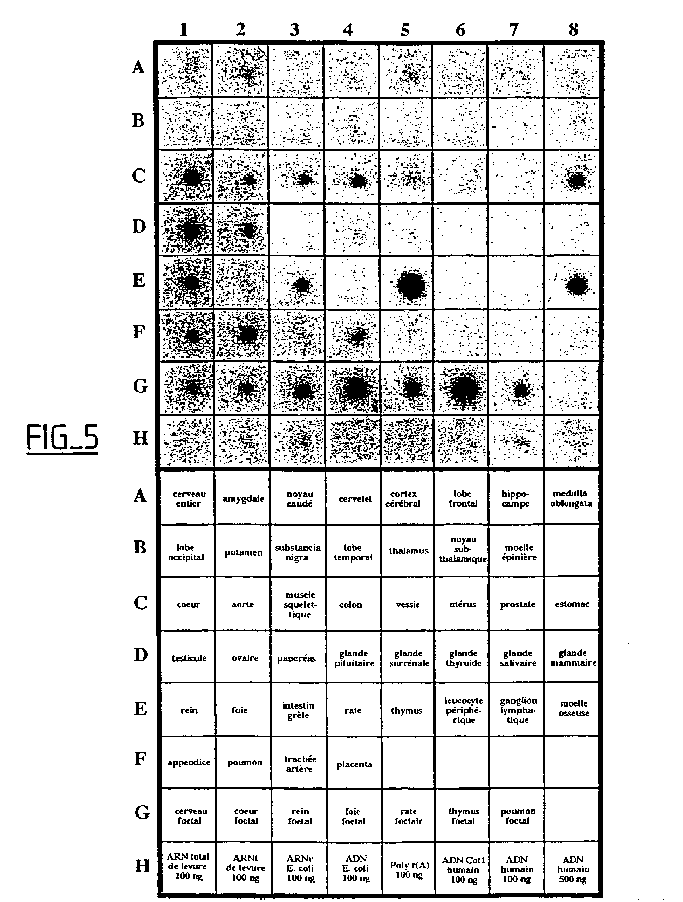

### Figure 5 : Expression de ICBP-59 dans divers tissus humains

Nous avons évalué le niveau d'expression de l'ARNm correspondant à ICBP59 sur un dot blot d'ARN comportant 50 tissus humains différents. Le blot a été hybridé pendant 16 heures à 68° C avec une sonde d'ADNc radioactive (³²P) de 679 pb dans une solution d'hybridation ExpressHyb (Clontech). Après lavages, on réalise une révélation par autoradiographie (exposition une semaine à 80°C). Les tissus présentant le plus haut niveau d'expression sont le thymus adulte et foetal, ainsi que la moelle osseuse adulte et le foie foetal.

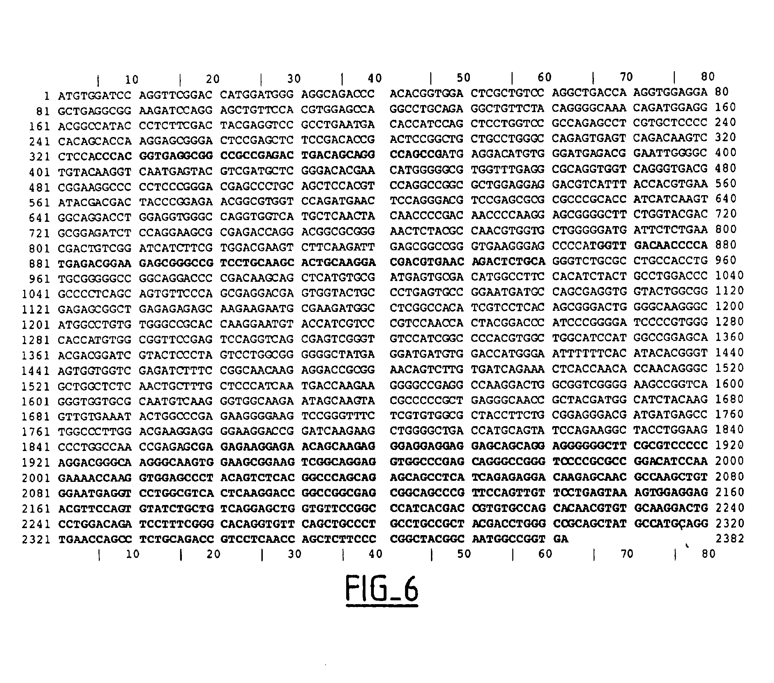

### Figure 6 : Séquence nucléotidique de ICBP90

L'ADNc codant pour ICBP90 comporte 2379 pb. Les portions de séquence indiquées en gras sont celles qui n'apparaissent pas dans les bases de données d'EST humains (human dbest). Les autres parties de la séquence existent dans diverses EST:
de 1 à 325 : EST n° AI083773.
de 367 à 865 : EST n° AA811055.
de 940 à 1857 : EST n° AA488755, EST n° AA129794 et
   EST n° AA354253.

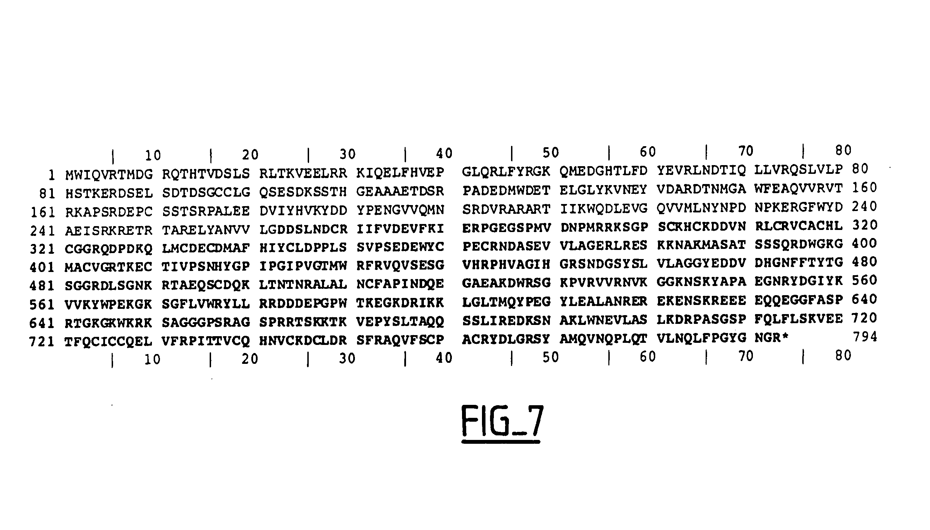

### Figure 7 : Séquence protéique de ICBP90

La séquence en acides aminés de ICBP90 est déduite par traduction de la séquence nucléotidique de la figure 6. ICBP90 comporte 793 résidus et présente un poids moléculaire théorique de 89,758 kDa. Le pKi est de 7,7. Les acides aminés indiqués en gras correspondent à ICBP-59.

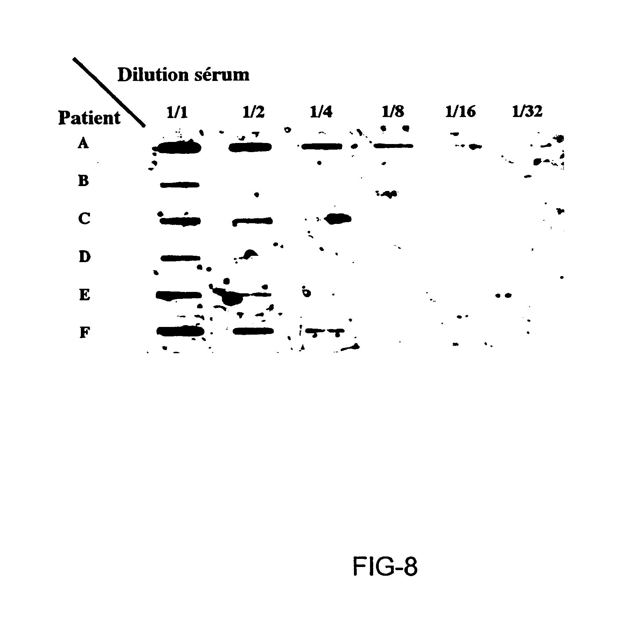

### Figure 8 : Détection de l'ICBP90 dans les sera de patients ayant des marqueurs sériques élevés de tumeurs solides.

Un volume de 2µl de sérum de chaque patient est dilué dans 1 ml de tampon PBS (Phosphate Buffer Saline 1X) contenant 0,1% de tween 20 suivi de dilutions croissantes réalisées dans le même tampon comme indiqué dans la figure. Un échantillon de 0,5 ml de chaque dilution est filtré sur la membrane de nitrocellulose à l'aide d'un appareil « Slot Blot BioRad ». La membrane est bloquée en présence de tampon PBS (contenant 0,1% de tween 20 et 5% de lait) pendant 1 heure à température ambiante. La protéine ICBP90 est révélée à l'aide de l'anticorps 1RC-1C10 (1ng/ml) et de l'anticorps secondaire (anti-souris couplé à la peroxydase dilué au 1/5000). Les bandes sont révélées par chimiluminescence par exposition pendant 10 secondes d'un film X-MAT (Kodak).

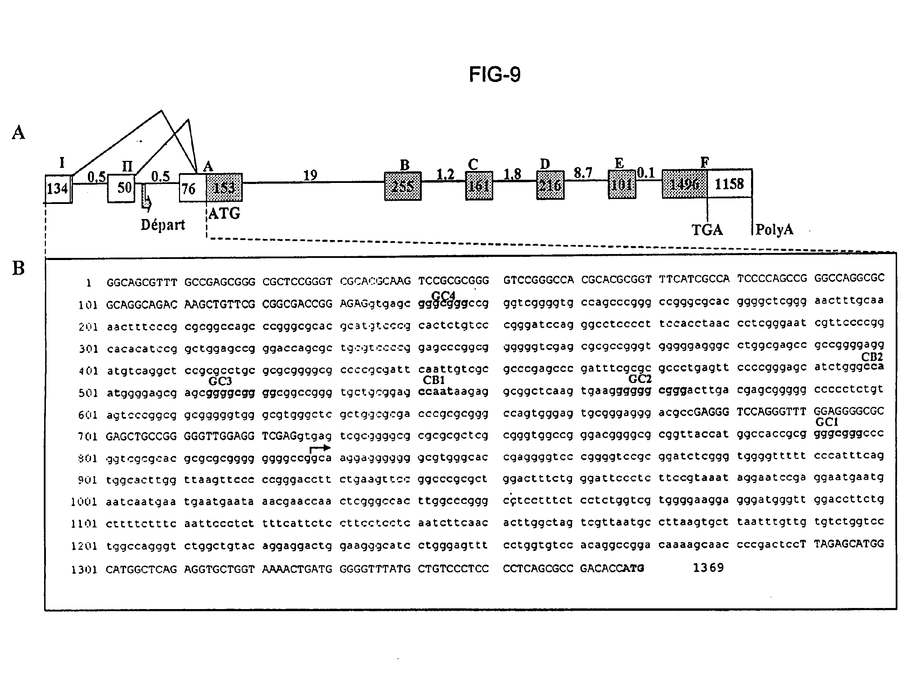

### Figure 9 : Organisation structurale du gène ICBP90.

**A.** Des exons représentés par des boîtes : les boîtes grises représentent des exons codants ; des boîtes blanches représentent des exons non-codants. La taille des exons est indiquée en pb dans chaque boîte, et les noms des exons sont identiques au-dessus des boîtes. Les introns sont mentionnés de manière schématique par des lignes fines et leur taille approximative est indiquée en pb. Un site putatif de démarrage de la transcription et un signal consensus de polyadénylation sont indiqués. L'ATG est le codon de début de traduction et TGA le codon d'arrêt de la traduction.

**B.** Séquence de la région 5' flanquante du gène ICBP90 (Seq ID N° 12) (Numéro d'accession Genbank N° AF 220 226 déposée le 30 décembre 1999). Les exons sont en majuscules et les introns en minuscules. Le codon de début ATG est en majuscules gras, les boites riches en GC (GC) et les boîtes CCAAT (CB) sont représentées en minuscules gras.

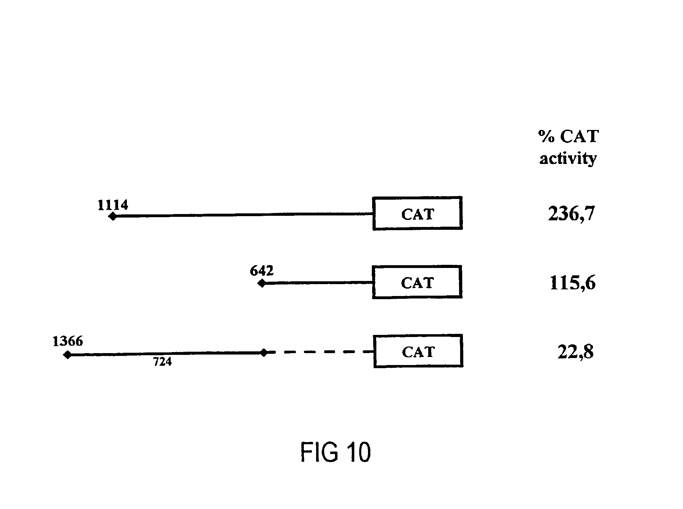

### Figure 10 : Analyse du promoteur d'ICBP90

Les séquences du promoteur d'ICBP90 ont été fusionnées à la séquence du gène « reporter » CAT dans le vecteur pBLCAT2 qui a ensuite été transfecté dans les cellules COS-1.

Une représentation schématique de ces constructions est représentée sur la gauche, avec le nombre se référant aux nucléotides en amont du codon d'initiation. Les activités CAT relatives des extraits cellulaires correspondant à l'induction de l'activité du promoteur TK minimal sont exprimées en pourcentage (à partir de trois expériences de transfection indépendantes) et sont indiquées sur la droite.

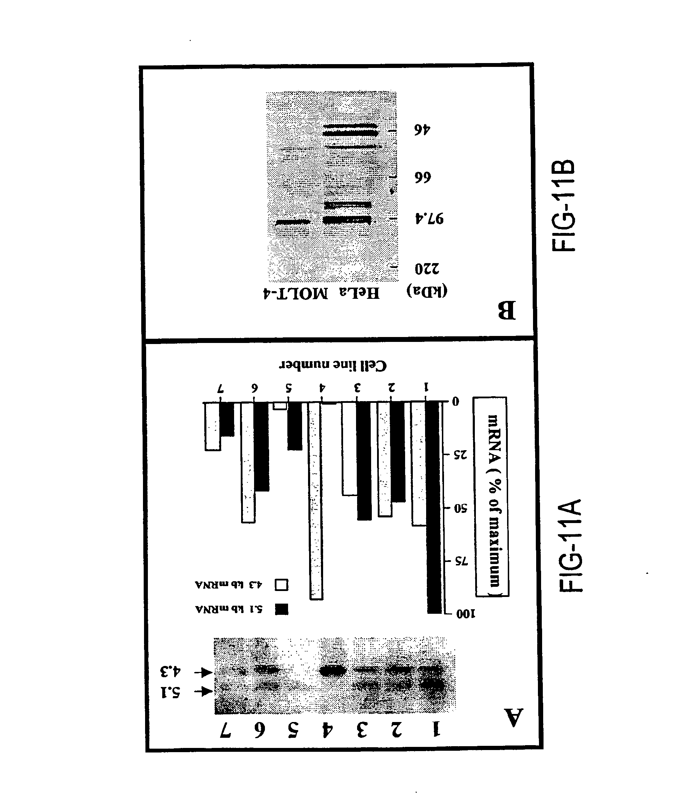

### Figure 11 : Analyse par Northern Blotting et Western Blotting de l'expression d'ICBP90.

A. L'hybridation Northern a été effectuée sur une membrane de Northern Blotting dont les dépôts d'ARN proviennent de lignées cellulaires cancéreuses de différents organes. Une sonde spécifique d'ICBP90, synthétisée par PCR, et marquée à la digoxigénine, a été utilisée pour la détection des ARNₘ d'ICBP90. Les tailles des ARNₘ sont mentionnées sur la droite de la ligne 7.

Les lignes 1 à 7 contiennent des ARN provenant respectivement de la lignée leucémique promyélocytaire HL-60, de Hela 53, de cellules K562 de leucémie myélogène chronique, de cellules de leucémie lymphoplastique MOLT-4, de cellules Raji du lymphome de Burkitt, de cellules SW480 d'adénocarcinome colorectal, et de cellules A549 de carcinome pulmonaire.

L'histogramme montre les taux d'expression des ARNₘ correspondant aux bandes de 5,1 kb et de 4,3 kb exprimés en pourcentage du taux d'expression de l'ARNₘ de 5,1 kb des cellules HL-60 (ligne 1, figure 11A).

### B. Analyse en Western Blotting de l'expression de ICBP90 dans les cellules MOLT-4 et Hela.

Des lysats de cellules totales de cellules Hela et MOLT-4 en prolifération ont été préparés. L'expression d'ICBP90 a été analysée en Western Blotting en utilisant l'anticorps 1RC1C-10.

### EXEMPLE 1 : MISE EN EVIDENCE D'UNE NOUVELLE PROTEINE DE LIAISON A LA SEQUENCE ICB

### 1.1. Construction reportrice pour le criblage de la banque

Le système du simple hybride est une technique puissante qui permet de détecter *in vivo* chez la levure l'interaction de protéines avec des séquences d'ADN spécifiques en criblant des banques d'ADNc. Ceci permet d'évaluer directement l'ADNc correspondant de la protéine à lier. Plusieurs études ont permis d'identifier la nouvelle protéine dans cette méthode. Ces méthodes décrivent très bien les protocoles utilisés (Inouye *et al*., 1994 ; Wang et Reed, 1993).

Brièvement, les oligonucléotides suivants ont été synthétisés 5'- AATTCG**ATTGG**TTCTG**ATTGG**TTCTG**ATTGG**TTCTT-3' et 5'-CTAGAAGAA**CCAAT**CAGAA**CCAAT**CAGAA**CCAAT**CG-3'. Ces nucléotides sont ensuites hybridés. Selon les instructions du fabricant (Clontech, Palo Alto, CA), la construction reportrice cible possède trois copies en tandem de la séquence ICB2 (ICB2X3). Comme mentionné plus haut, une copie de ICB2 est soulignée et les séquences CCAAT sont représentées en gras. Pour déterminer la spécificité de liaison des protéines à la boîte ICB, les oligonucléotides suivants qui contiennent trois copies en tandem de la boîte GC1 (GC1X3) et également présents dans le promoteur ont été synthétisés et hybridés:
5'- AATTC**GGGGCGGG**GCC**GGGGCGGG**GCC**GGGGCGGG**GCT-3'
5'- CTAGAG**CCCCGCCCC**GG**CCCCGCCCC**GG**CCCCGCCCC**GG-3'

Les fragments d'ADN cible résultant sont clonés dans le polylinker d'un plasmide intégratif pHISi-1 (Clontech) par ligation des extrémités cohésives au niveau du site EcoRI et XbaI, en amont du promoteur minimal du gène *his*3. La souche de levure YM4271 (Clontech) est utilisée pour la transformation et des colonies de levure ayant intégrées le plasmide dans leur génome sont sélectionnées sur un milieu synthétique Dropout ne contenant pas d'histidine. Deux clones ont été isolés : un pour ICB2 et un pour la boite GC 1.

### 1.2. Criblage de la banque

Une banque d'ADNc de la lignée cellulaire Jurkat clonée au site d'EcoRI du polylinker en aval de GAL4-AD du vecteur pGAD10 (Clontech) est utilisée pour le criblage selon les instructions du fabricant. Des clones positifs sont sélectionnés puis cultivés sur un milieu sélectif déplété en histidine et en leucine. L'ADN plasmidique de ces clones est récupéré et introduit par électroporation dans des bactéries *Escherichia coli* XL1-blue. Le séquençage des inserts a été réalisé sur une matrice d'ADN plasmidique purifiée à partir d'une culture d' 1,5 ml utilisant un kit de mini préparation (Bio-Rad, Hercules, CA, USA). Une banque d'ADNc de thymus humain cloné dans λgt10 (Clontech) a été criblée par hybridation sur une plaque, pour récupérer un ADNc codant la partie N-terminale de la protéine.

### 1.3. Découverte de ICBP-59

Les ADNc de quatre clones ayant rempli les critères de sélection du système simple hybride ont été séquencés, et les profils analysés à l'aide de bases de données informatiques (Genbank, EMBL, PDB, Swissprot) afin de déterminer la nature des protéines codées. Deux clones correspondent à des protéines ribosomales (hRS12 et hRS4), un à une sérine-thréonine kinase (STPLK-1) et le quatrième à une protéine humaine d'un poids moléculaire théorique de 59 kDa (calculé à partir de la séquence traduite) et non répertoriée.

Les ADNc, codant pour hRS4, hRS12 et ICBP-59 et obtenus par digestion par EcoRI des clones positifs obtenus dans le vecteur pGAD10, ont été clonés au site EcoRI du vecteur d'expression pGEX-4T-1 (Pharmacia). Les ADN recombinants sont ensuite transformés dans une souche d'*Escherichia coli* adaptée (BL21). 500 ml de culture du clone sélectionné ont été utilisés lorsque une densité optique de 0,5 a été atteinte. La surexpression des protéines d'intérêt a été induite par l'IPTG (1mM) pendant 2h à 37° C. Le vecteur pGEX-4T-1 conduit à l'obtention de grandes quantités de protéines sous forme fusionnée à la glutathion-S-transférase (GST). Les protéines de fusion avec la glutathione-S-transférase (GST) sont ensuite purifiées en utilisant des billes de sépharose couplé au glutathion (Pharmacia) suivi par une coupure durant la nuit avec de la thrombine (0,05 U/ml) à 4° C (Pharmacia).

Pour tester l'aptitude de la protéine de poids moléculaire de 59 kDa à lier spécifiquement les boites ICB1 et/ou ICB2, trois copies en tandem de ICB2 (ICB2X3, séquences décrites précédemment) ont été marquées au niveau terminal au phosphore ³²P en utilisant la polynucléotide kinase T4 (New England Biolabs) et du [γ³²P]ATP (160 mCi/mmol, ICN Irvine, CA, USA). Pour examiner la spécificité de la liaison, des oligonucléotides contenant seulement une copie de la boite CCAAT ont été synthétisés :
ICB1: 5'-AGTCAGGG**ATTGG**CTGGTCTG-3';
   5'- CAGACCAG**CCAAT**CCCTGACT-3'
ICB2: 5'-AAGCTACG**ATTGG**TTCTTCTG-3';
   5'-CAGAAGAA**CCAAT**CGTAGCTT-3'.

La protéine ICBP-59 purifiée (1 µg) est incubée avec 1 ng d'oligonucléotide marqué à son extrémité terminale par du phosphore ³²P dans 12% de glycérol, 12 mM d'HEPES-NaOH (pH 7,9), 60 mM KCI, 4 mM Tris-HCl (pH 7,9), 100 ng BSA, 0,6 mM DTT et 100 ng de poly(dI/dC) dans 20 µl (Inouye et al., 1994). Après 30 minutes d'incubation à température ambiance, le mixte réactionnel est chargé sur des gels de polyacrylamide à 6%. Dans les expériences de compétition, la quantité indiquée d'oligonucléotides non marqués est ajoutée au mixte réactionnel 10 min avant l'addition de protéines. Pour examiner les propriétés de liaison de l'ICBP90 à la boite ICB2, le même protocole est utilisé à la différence que l'oligonucléotide marqué contient seulement une copie de la séquence CCAAT telle que décrite ci-dessous:
ICB2: 5'-ATAAAGGCAAGCTACG**ATTGG**TTCTTCTGGACGGAGAC-3'
   5'-GTCTCCGTCCAGAAGAACC**AAT**CGTAGCTTGCCTTTTAT-3'

La spécificité de liaison est étudiée en utilisant un nucléotide non marqué contenant une boite GC du promoteur du gène de la topoisomérase IIα humaine :
5'-GAATTCGAGGGT**AAAGGGGCGGGG**TTGAGGCAGATGCCA-3'
5'-TGGCATCTGCCTCAA**CCCCGCCCC**TTTACCCTCGAATTC-3'.

Ces expériences de retard de migration sur gel d'acrylamide, nous ont permis de mettre en évidence que la nouvelle protéine humaine de 59 kDa est capable de lier une séquence d'ADN de type ICB, et ce de manière spécifique. Nous avons appelé cette protéine ICBP-59 (pour Inverted CCAAT Box Binding Protein of 59 kDa).

### EXEMPLE 2 : CARACTERISATION DE LA PROTEINE ICBP90

### 2.1. Synthèse d'anticorps

Les anticorps monoclonaux de souris sont synthétisés dans notre Laboratoire par injection de la protéine ICBP-59 par les méthodes traditionnelles (Brou *et al*., 1993) ; la protéine a été au préalable purifiée par un système de fusion GST. Deux anticorps monoclonaux de 1RC1C-10 et 1RC1H-12 ont été sélectionnés pour leur performance à détecter la protéine endogène correspondant à la protéine ICBP-59 à la fois dans des expériences de Western blotting et dans des expériences d'immunocytochimie. Avant utilisation, les anticorps sont purifiés sur colonne DEAE-cellulose (DE52, Whatmann) à partir des liquides d'ascites.

### 2.2. Mise en évidence de la protéine endogène par Western Blotting

Afin de détecter la protéine endogène correspondant à ICBP-59, nous avons dans un premier temps utilisé 1RC1C-10 en Western blot (0,4 µg/ml d'anticorps monoclonal 1RC1C-10) sur des extraits nucléaires de cellules HeLa en situation de prolifération et de confluence (Figure 1). Les cellules COS-1 et HeLa sont cultivées tel que décrit précédemment (Brou et al., 1993 ; Gaub *et al.*, 1998; Rochette-Egly *et al.*, 1997). Les cellules MOLT-4 sont cultivées dans de l'air à 100% dans du RPMI supplémenté par 10% de sérum de veau fetal. Les fibroblastes pulmonaires humains en culture primaire sont préparés et cultivés dans du DMEM/F12 tel que décrit précédemment (Kassel et al., 1998). Des extraits nucléaires de cellules Jurkat ont été achetés chez Sigma alors que ceux de MOLT-4 et HL60 ont été préparés tel que décrit auparavant (Lavie *et al.*, 1999). Les cellules HeLa en phase de croissance et les fibroblastes pulmonaires humains sont obtenus par déplétion de la culture en sérum pendant 30 h suivi par la réintroduction pendant 16 heures par 10% de sérum de veau fétal (v/v). La prolifération est arrêtée lorsque la confluence atteint 60 à 70%. Les cellules arrêtées à confluence (confluence de 100%) sont obtenues de manière concomitante en omettant l'étape de déplétion en sérum. Pour ces deux types cellulaires, des lysats cellulaires bruts sont préparés en récoltant les cellules dans du PBS (phosphate buffer saline) suivi d'une étape de sonication. Pour les expériences d'immunotransfert des lysats de cellules totales et des extraits nucléaires sont chargés sur des gels de polyacrylamide SDS à 8% pour réaliser une électrophorèse en une dimension. Les protéines sont transférées sur des membranes de nitrocellulose bloquées avec un réactif de blocage 10% (Roche Molecular Biochemicals, Mannheim, Germany) et incubées avec l'anticorps monoclonal purifié (1RC1C-10) à la concentration de 0,5 µg/ml. Un anticorps anti-souris de mouton couplé à la phosphatase alcaline (fragments Fab, Roche Molecular Biochemicals) est utilisé à une dilution de 1/2 500. Des signaux sont détectés en utilisant le chlorure de 4-nitro blue tetrazolium/5-bromo-4-chloro-3-indolyl-phosphate comme substrat.

Ces expériences montrent que la protéine endogène présente un poids moléculaire apparent d'environ 97 kDa. En outre, on observe que les formes de la protéine varie en fonction de la nature tumorale ou non-tumorale des cellules ainsi que de l'état de confluence ou de prolifération des cellules. En effet, dans les pistes correspondant aux extraits de cellules HeLa, on observe une bande majeure à 97 kDa ; pour les cellules heLa en prolifération, des bandes supplémentaires de tailles inférieures à 97 kDa apparaissent (piste 2). Dans les fibroblastes pulmonaires humains à confluence, la protéine endogène n'est pas exprimée (piste 3) et apparaît lorsque les cellules se mettent à proliférer (piste 4). Ces observations suggèrent que la protéine endogène ICBP90 est un marqueur de prolifération cellulaire pour des cellules normales (fibroblastes) tandis que pour les cellules tumorales elle serait un marqueur quelque soit le stade cellulaire.

L'utilisation de l'anticorps monoclonal dans des expériences d'immunoprécipitation sur des extraits de protéines nucléaires, suivies d'un Western blot, conduit de la même manière à la mise en évidence d'une protéine de 97 kDa (Figure 2).

Les résultats obtenus en Western blot, aussi bien pour les extraits de protéines nucléaires que pour les immunoprécipitations, montrent que la protéine de 59 kDa isolée à l'aide du système simple hybride ne constitue qu'un fragment de la protéine endogène humaine correspondante, en l'occurrence le fragment C-terminal à partir du résidu D263. Il nous a donc fallu entreprendre un nouveau criblage de banque d'ADNc.

### 2.3. Analyse en Dot Blot d'ARN de multiples tissus humains

Afin de choisir une banque nous donnant le plus de chance possible d'isoler la protéine complète, nous avons voulu identifier un tissu humain exprimant l'ARN messager (ARNm) correspondant en quantité importante. A l'aide d'une sonde d'ADNc recouvrant une partie de la séquence de ICBP59 et marquée au ³²P, nous avons testé l'expression de l'ARNm d'intérêt dans 50 tissus humains différents sur un dot blot d'ARN. Brièvement, une sonde longue de 678 paires de bases correspondant à la séquence en acides aminés 269 à 500 de ICBP90 a été synthétisée par PCR en utilisant de la Taq polymérase (Sigma, St Louis, MO, USA). La sonde marquée par random priming en utilisant du dCTP-α ³²P est purifiée sur colonnes Sephadex G50 (Pharmacia, Uppsala, Suède).

Un dot blot d'ARN de multiples organes contenant de l'ARN poly(A)⁺ de 50 tissus humains différents est hybridé 20 heures dans des conditions de forte stringence dans un milieu ExpressHyb (Clontech) à 68° C avec une sonde marquée au ³²P. Des lavages à haute stringence sont réalisés dans du 0,1 x SSC, O,1% SDS à 68° C (De Vries et al., 1996).

Les réultats obtenus (figure 5) montrent que les tissus exprimant le plus fortement l'ARNm de la protéine ICBP-59 sont le thymus adulte et foetal, ainsi que la moelle osseuse adulte et le foie foetal. Pour isoler la protéine entière, notre choix s'est donc porté sur une banque d'ADNc de thymus adulte.

### 2.4. Criblage de la banque et clonage de ICBP90

Le criblage de la banque nous a permis d'obtenir plusieurs clones d'environ 4000 paires de bases (pb) comportant un cadre de lecture ouvert de 2379 pb (Fig.6). Cette séquence code pour une protéine de 793 acides aminés (Fig.7) dont le poids moléculaire théorique (calculé à partir de la séquence traduite) est de 89,758 kDa. Nous avons appelé cette protéine ICBP90 (pour Inverted CCAAT Box Binding Protein of 90 kDa) par analogie avec l'appellation utilisée pour la protéine initiale de 59 kDa.

L'ADNc ICBP90 (2379 bp) a été synthétisé par PCR en utilisant l'ADN polymérase Deep Vent (New England Biolabs, Beverly, MA, USA) et les oligonucléotides utilisés au cours de cette réaction de PCR étaient voisins du site de EcoRI. Le produit de réaction a été par la suite souscloné dans un vecteur pGEX-4T-1 (Pharmacia) pour l'expression de la protéine de fusion GST dans BL21. La surexpression est induite par IPTG (1mM) pendant 4h à 25° C. La protéine ICBP90 est ensuite purifiée.

### 2.5. Immunocytochimie et immunohistochimie.

L'observation directe de la protéine ICBP90 sur des cellules et tissus a été également mise en oeuvre.

Des cellules COS-1 ont été transfectées comme décrit précédemment (Brou *et al.*, 1993 ; Gaub et al., 1998) avec le vecteur pSG5 (Stratagene, La Jolla, CA) dans lequel l'ADNc de ICBP90 (2379 bp) a été sous-cloné dans le site de restriction EcoRI. L'ADNc est synthétisé par réaction de polymérisation en chaine (PCR) utilisant la polymérase Deep Vent (New England Biolabs) et des oligonucléotides flanquants le site de restriction EcoRI. La construction plasmidique est vérifiée par séquençage. L'immunomarquage des cellules HeLa et des cellules COS-1 transfectées est réalisé tel que décrit précédemment (Brou *et al*., 1993) avec respectivement les anticorps monoclonaux 1RC1C-10 et 1RC1H-12. Un marquage indirect à l'immunopéroxidase de ICBP90 et de topoisomérase IIα est réalisé comme décrit précédemment (Rio *et* al., 1987, Devys *et al*., 1993). Les appendices humains ont été inclus dans la paraffine et fixés dans du formalin 10% tamponé (Sigma). Des coupes sériées (3 µm) sont incubées durant la nuit à température ambiante avec l'anticorps 1RC1C-10 et avec l'anticorps anti-topoisomérase IIα (NeoMarkers, Union City, CA, USA). Des anticorps liés de manière spécifique sont visualisés par un complexe utilisant la streptavidine biotine (LAB/LSAB method, Dako LSAB2 System kit ; DAKO, Carpinteria, CA, USA).

En immunocytochimie, l'anticorps 1RC1C-10 marque le noyau des cellules HeLa tandis que le nucléole et l'ensemble du cytoplasme ne sont pas marqués (Figure 3). En immunohistochimie, des coupes en paraffine d'appendice humain montrent un marquage localisé essentiellement dans des zones de prolifération cellulaire (Figure 4). En effet, les cellules marquées sont logées dans les cryptes glandulaires (CG) ainsi que dans les zones germinatives (Ger). Un marquage identique est obtenu lorsqu'on utilise un anticorps anti-topoisomérase IIα qui est une enzyme uniquement exprimée dans des cellules en prolifération (résultats non illustrés).

### 2.6. Recherches BLAST et prédiction de domaines

Les études sur BLAST en ligne ont été réalisées à partir des informations du National Center for Biotechnology Information au National Institute of Health (Bethesda, MD, USA). SCANPROSITE et PROFILESCAN sont utilisés pour l'analyse protéique (Infobiogen, Villejuif, France).

ICBP90 comporte un domaine « ubiquitin-like » dans ses 80 premiers acides aminés, deux sites de localisation nucléaires potentiels dans la partie C-terminale et deux domaines en doigt de zinc (« zinc-finger »), dont l'un serait impliqué dans la liaison à l'ADN et l'autre dans des interactions protéine-protéine. Plusieurs sites potentiels de phosphorylation par la protéine kinase C, la caséine kinase II ainsi que par une tyrosine kinase sont également présents.

La production et la purification de ICBP90 à l'aide du système de fusion GST (même procédé que celui utilisé pour ICBP-59) nous a finalement permis de tester la capacité de la protéine complète à lier les séquences d'ADN de type ICB. Son comportement est en tous points identique à celui observé pour ICBP-59.

En définitive, nous avons isolé une nouvelle protéine humaine que nous avons appelée ICBP90 pour les raisons évoquées ci-dessus. Son poids moléculaire théorique est de 89,758 kDa et son poids moléculaire apparent sur gel d'acrylamide est de 97 kDa. Cette protéine est non seulement localisée exclusivement dans le noyau des cellules humaines, mais elle présente également la capacité à lier des séquences d'ADN de manière spécifique, en l'occurrence des séquences de type CCAAT. Pour ces raisons, nous pensons que ICBP90 a la possibilité de moduler l'expression des gènes dont le promoteur est pourvu de boîtes CCAAT, éventuellement en position inversée (ICB). Le gène de la topoisomérase IIα humaine qui nous intéresse plus particulièrement, et qui comporte cinq séquences ICB dans son promoteur, nous semble être une des cibles privilégiées de ICBP90.

Ces expériences ont permis de mettre en évidence les caractéristiques remarquables de l'anticorps 1RC1C-10 qui ne marque uniquement que les cellules en prolifération dans le cas de cellules non cancéreuses ; il marque les cellules cancéreuses en prolifération ou en quiescence ; il est utilisable dans 4 techniques différentes (Western blotting, immunocytochimie, immunohistologie, immunoprécipitation) ; il possède une très bonne affinité et permet d'utiliser une dilution de 1/150 000 en immunohistochimie (i.e. 13 ng/ml) ; enfin, son utilisation ne génère quasiment pas de bruit de fond.

Les applications futures de 1RC1C-10 se situent en premier lieu dans les domaines du diagnostique et de la recherche fondamentale. Pour le diagnostique en anatomo-pathologie par exemple, il serait tout à fait possible de rendre compte de l'état prolifératif d'un tissus cancéreux donné. Concernant la recherche fondamentale, des investigations sont en cours dans notre laboratoire afin de déterminer la contribution exacte de ICBP90 dans les mécanismes de prolifération des cellules normales et des cellules cancéreuses. Or, pour l'étude de l'expression de ICBP90 en fonction du cycle cellulaire, de sa localisation nucléaire précise et de son interaction avec d'autres protéines cellulaires, l'utilisation de l'anticorps sera incontournable.

Pour l'instant nous n'avons pas étudié l'expression de l'ICBP90 en fonction du cycle cellulaire. Néanmoins, dans le cas ou des lignées de cellules cancéreuses sont confluentes ou lorsqu'elles sont en prolifération nous ne pouvons détecter de différences significatives de l'expression de l'ICBP90 (Fig. 1) du moins en ce qui concerne la forme à 97 kDa. Par contre, dans des cellules confluentes non cancéreuses (cellules musculaires lisses bronchiques humaines) l'expression de l'ICBP90 est difficilement détectable (résultats non illustrés). Ceci est confirmé sur les coupes histologiques où aucune cellule en quiescence n'est marquée par l'anticorps. Il est par conséquent possible que l'ICBP90 soit exprimée quelle que soit la phase du cycle cellulaire dans des cellules cancéreuses alors que son expression varierait en fonction de chaque phase dans des cellules non cancéreuses. Ceci rend donc l'utilisation de l'anticorps extrêmement intéressante, en ce sens que nous aurions à disposition un marqueur de la prolifération cellulaire de tissus cancéreux qui ne dépendrait pas de la phase du cycle cellulaire contrairement à d'autres marqueurs de prolifération cellulaire tels que le Ki-67, la topoisomérase IIα, la cycline E et la cycline B1. En effet, la fin de la phasé S est caractérisée par une très faible expression de Ki-67, la cycline E marque les cellules en fin de phase G 1 jusqu'au milieu de la phase S et la cycline B1 marque les cellules en phase G2/M (pour revue Darzynkiewicz et al., 1994). Par ailleurs, il a été montré que PCNA (Proliferating Cell Nuclear Antigen) surestime le nombre de cellules en prolifération dans certains types de tissus (Roskell and Biddolph, 1999).

ICBP90 joue un rôle important dans la prolifération cellulaire en régulant l'expression de gènes tels que celui de la topoisomérase IIα. Différentes stratégies visant à bloquer l'action de cette protéine doivent permettre de modifier la prolifération cellulaire. Ainsi, l'utilisation de l'anticorps 1RC1C-10 ainsi que l'utilisation de peptides mimant l'interaction ADN/ICBP90 sans pour autant engendrer d'effet physiologique subséquent constitue une possibilité intéressante. Le design de ses peptides s'inspirerait directement de la séquence protéique de ICBP90 que nous avons décrite. Une forme tronquée correspondant à ICBP59 pourrait par exemple être un des premiers candidats.

Le blocage pur et simple de l'expression de ICBP90 dans le but d'éliminer complètement son influence sur les gènes et par extension sur la prolifération cellulaire peut être envisagé ; il peut se faire soit par une approche classique en obtenant des inhibiteurs de la protéine, soit en utilisant une approche plus moderne correspondant à la technique d'interférence par l'ARN double brin (RNA interference ou RNAi) telle que décrit récemment par Kennerdell & Carthew (1998).

### EXEMPLE 3: ISOLEMENT ET CARACTERISATION DU GENE ICBP90

### 3.1. Matériels et méthodes

### 3.1.1. Construction et criblage d'une banque génomique placentaire humaine

Après digestion partielle avec l'enzyme MboI, l'ADN génomique placentaire a été fractionné en fonction de la taille sur un gradient de 10 à 40% de sucrose. Des fragments d'ADN de 15 kb ont été ligués dans un vecteur λGEM12 préalablement digéré par BamHI (Promega, Madison WI, USA). Après empaquetage, les particules de phages λ ont été titrées sur des cellules TAP 90. La banque génomique contient 3.10⁶ unités formant des plaques (plaques forming units, pfu). 10⁶ clones ont été étalés pour l'analyse. Une sonde de 620 pb correspondant à une extrémité 5' terminale de l'ADNc de ICBP90 utilisée pour le criblage a été marquée au α³²P-dCTP par une méthode d'amorçage aléatoire (random priming) (Sambrook et al., 1989). La sonde marquée est utilisée selon un protocole classique d'hybridation sur plaque pour cribler la banque génomique (Sambrook et al., 1989). L'hybridation a été réalisée à 68°C dans du 5X SSC (15 mM NaCl, 1,5 mM citrate de sodium pH 7,0), 5 X de solution Denhardt, 100 µg / ml d'ADN de sperme de saumon et 0,1% de SDS, suivi par 30 minutes de lavages dans du 2X SSC, 0,1% SDS à température ambiante.

Deux étapes de criblage ont été réalisées pour purifier un clone positif. Le clone positif a ensuite été digéré avec l'enzyme NotI et deux fragments de 6 et de 10 kb ont été sous-clonés dans le vecteur pBluescript-SK⁺ (Stratagène, La Jolla CA, USA) selon un protocole standard (Sambrook *et al*., 1989).

### 3.1.2. Criblage de la banque d'ADNc de thymus humain

Une banque λGT10 d'extrémité 5' d'ADNc de thymus humain (Clontech, Palo Alto, CA, USA) a été criblée par hybridation sur plaque en utilisant la sonde d'ADNc de 679 pb synthétisée telle que dans le paragraphe relatif à l'analyse par Northern Blotting. Des signaux ont été détectés en utilisant du chlorure de 4-nitro-bleu-tétrazolium et de 5-bromo-4-chloro-3-indolyl-phosphate comme substrat.

### 3.1.3. Réaction de polymérisation en chaîne (PCR) sur l'ADN génomique placentaire

L'ADN génomique placentaire a été préparé selon une méthode conventionnelle (Sambrook *et al*., 1989). Pour la région 5' du gène ICBP90, les inventeurs ont utilisé le kit PCR Advantage^{®}-GC genomic de Clontech qui est adapté aux régions riches en GC de l'ADN génomique. Pour couvrir les régions 3'-flanquantes, la Taq polymérase (Sigma, St Louis, MO, USA) et son tampon correspondant ont été utilisés. Les réactions ont été réalisées selon les instructions du fabricant en utilisant 250 ng d'ADN génomique placentaire comme matrice dans un volume final de 50µl. Afin d'obtenir l'amplification des introns de longueur 19 kb et 8,7 kb le système PCR Expand™ 20kb^{plus} (Roche Diagnostics, Mannheim, Germany) a été utilisé.

La réaction a été réalisée dans 100µl en utilisant 125 ng d'ADN génomique placentaire par réaction.

### 3.1.4. Constructions plasmidiques et essais CAT

Une série de différents fragments ont été obtenus par PCR dans la région 5' flanquante du gène ICBP90 en utilisant des amorces de 20 nucléotides afin d'obtenir les constructions décrites dans la figure 10. Celles-ci contiennent un site de restriction BamHI et l'ADN génomique placentaire humain a été utilisé comme amorce. Les produits PCR ont été digérés et sous-classés en amont du gène reporter chloramphénicol acétyl transférase (CAT) d'un vecteur contenant le promoteur minimal de la thymidine kinase (pBICAT2). Les constructions plasmidiques ont été vérifiées par séquençage. Des cellules COS-1 ont été cultivées dans un milieu Dulbecco modifié par Eagle (DMEM) supplémenté avec 5% de sérum de veau fétal. Après l'étalement, les cellules ont été transférées avec les différentes constructions plasmidiques (5 µg) en utilisant la technique de co-précipitation au phosphate de calcium (Banerji *et al*., 1981)). Les analyses d'expression de la CAT ont ensuite été réalisées comme décrit ailleurs (Goetz *et al*. (1996)).

### 3.1.5. Localisation chromosomique du gène ICBP90

Des chromosomes métaphysiques ont été préparés à partir de leucocytes humains du sang périphérique selon les protocoles standards (Haddad *et al*. (1988)). Brièvement, une sonde de 10 kb correspondant à un fragment 5' terminal du clone de 16 kb isolé à partir du criblage de la banque d'ADN génomique placentaire, a été marquée avec de la biotine-16-dUTP (Roche Diagnostics) par « nick-translation ». La sonde est ensuite précipitée avec un excès (50X) d'ADN humain Cot-1 (Life Technologies, Rockville MD), resuspendu dans 50% de formamide, 1X SSC, pré-hybridé pendant 2 heures à 37°C puis hybride sur la nuit à 37°C. La détection est réalisée en utilisant de l'avidine-FITC (Vector Laboratories, Burlingam CA). Les chromosomes ont été contre-colorés avec du 4'-6-diamino-2-phénylindole (Sigma).

### 3.1.6. Analyse de Northern blotting et de Western Blotting

Une membrane de Northern Blotting contenant 2 µg d'ARN polyA⁺ par ligne, provenant de 7 lignées cellulaires humaines cancéreuses différentes (Clontech) a été préhybridée dans du Express Hyb (Clontech) puis hybridée avec la sonde spécifique de ICBP90 dans du Express Hyb à 68°C pendant deux heures. La sonde double-brin marquée à la digoxigénine a été préparée par amplification PCR d'un fragment de 676 pb à partir de l'ADNc d'ICBP90 (nucléotides 806 à 1 485 ; Numéro d'Accession Genbank AF 129 507) selon les instructions du fabricant (Roche Diagnostics).

Après purification au travers d'une colonne de chromatographie Micro Bio-Spin 30 (Bio-Rad, Hercules, CA), la sonde spécifique ICBP90 (5 ng/ml) a été chauffée à 95°C pendant 15 minutes puis refroidie dans la glace avant l'addition de la solution d'hybridation. Les lavages après l'hybridation ont été réalisés deux fois dans du 2X SSC, 0,1% SDS (30 minutes par lavage à température ambiante), puis deux fois dans du 0,1X SSC, 0,1% SDS (30 min. par lavage à 68°C). La membrane a été traitée avec la solution A (0,1 M acide malique, 0,15 M de NaCl à pH 7,5) puis bloquée par incubation avec 1% d'agent bloquant (Roche Diagnostics) dans le tampon A pendant 30 minutes à température ambiante.

Un anticorps conjugué à la phosphatase-alcaline dirigé contre la digoxigénine (fragment Fab, Roche Diagnostics) a été ajouté (150 mU/ml) puis incubé pendant 30 minutes à température ambiante. La membrane a ensuite été lavée deux fois avec la solution A puis équilibrée dans du 0,1 M tris-HCl, 0,1 M NaCl, pH 9,5. Pour la détection par chémiluminescence, les inventeurs ont utilisé l'agent Disodium 3-(4-méthoxyspiro{1,2 dioetane-3,2'-(5'-chloro) tricyclo [3.3-1.1^{3.7}] décan }-4-yl) phényl phosphate^{®} (Roche Diagnostics) selon les instructions du fabricant. Les bandes d'ARNₘ ont été quantifiées en utilisant le logiciel NIH image 1.62 et exprimées en pourcentage de la bande d'ARNₘ la plus abondante (c'est-à-dire la bande de 5,1 kb des cellules HL-60).

L'analyse en Western Blotting a été réalisée comme décrit ailleurs (Hopfner *et al*. (2000)). Les signaux ont été détectés en utilisant le chlorure de 4-nitro-blue tétrazolium / le phosphate de 5-bromo-4chloro-3-indolyl comme substrat.

### 3.1.7. Outils de recherche d'alignement local de base, prédictions de sites de démarrage de la transcription et de signal polyA

Des recherches d'alignement local de base ont été réalisées via le Centre National d'Information en Biotechnologie au National Institute of Health (Bethesda, MD, USA). Le criblage d'une banque de facteurs de transcription avec le programme d'ordinateur Mat Inspector, les prédictions de sites de démarrage de la transcription (TSS) avec Neural Network, ainsi que la prédiction de signal polyA, ont été réalisés via le Baylor College of Medicine (Reese *et al*. (1996)).

### 3.2. Résultat

### 3.2.1. Isolement et caractérisation du gène de l'ICBP90

Une banque d'ADN complémentaire de placenta humain cloné dans le phage lambda GEM 12 a été criblée à l'aide d'une sonde d'ADN. Le criblage a conduit à la purification d'un seul clone positif ayant un insert de 16 kb. L'analyse de la séquence a permis de déterminer qu'il contenait une séquence intronique longue de 10 kb et contenant 3 exons (appelé B, C et D dans la figure 9A). Tous les autres criblages, incluant notamment ceux qui ont été réalisés par PCR sur des banques de BAC (Bacterial Artificial Chromosome) ou de YAC (Yeast Artificial Chromosome), n'ont pas permis d'isoler d'autres clones positifs. Par conséquent, nous avons décidé de déterminer le reste de l'organisation du gène par PCR directement sur de l'ADN génomique de placenta humain. La plus grande difficulté fut d'obtenir le côté 5' de l'intron de 19 kb. Ainsi, des amorces ont été choisies dans l'exon A (amorce sens) et dans le côté 5' du clone de 16 kb (amorce anti-sens). L'exon E et l'intron de 8,7 kb ont été amplifiés en utilisant une amorce sens dans l'exon D et l'amorce anti-sens dans l'exon F. Finalement, la séquence complète de l'exon F jusqu'au signal de poly-adénylation a été déterminée en utilisant une amorce sens choisie dans le début de l'exon F et l'amorce anti-sens dans le côté 3' d'une EST (référence dans GenBank n° AW297533) homologue a la séquence du gène de l'ICBP90. La séquence complète du gène de l'ICBP90 montre qu'il est composé de 6 exons codants dont la taille varie de 100 paires de bases à 3453 paires de bases. La plupart des jonctions exons/introns répondent aux séquences consensus pour les sites accepteurs et donneurs d'épissage. Une séquence consensus de poly-adénylation (AATAAA) a été trouvée dans la région 3', c'est-à-dire 1152 nucléotides après le codon stop dans la figure 9A.

### 3.2.2 La région 5' du gène de l'ICBP90

Le criblage d'une banque d'ADN complémentaire de thymus humain cloné dans le phage lambda gt 10 a conduit à l'obtention de deux populations de cDNA qui se distinguent l'une de l'autre dans leur région 5', exactement 10 paires de bases en amont du codon d'initiation, c'est-à-dire dans la région 5' non traduite. Ces deux populations de cDNA prédisent l'existence de deux exons alternatifs en 5' appelés exon I et exon II (Figure 9A). Nous avons observé que les exons I et II sont reliés à un site d'épissage alternatif interne de l'exon A. De plus, nous avons trouvé dans une base de données un EST (référence dans GenBank n° AI084125) correspondant aux nucléotides 1290 à 1356 (Figure 9B). Les positions de ces deux exons et de l'EST à l'intérieur du locus ont été déterminées par PCR. Pour cela nous avons utilisé des amorces correspondant aux 18 premiers nucléotides de chaque exon et une amorce anti-sens choisie dans le premier exon traduit (exon A). Cette stratégie nous a permis de reconstruire la région 5' telle qu'elle est représentée dans les figures 9A et 9B, avec l'exon I correspondant aux nucléotides 1 à 134 et l'exon II correspondant aux nucléotides 676 à 725. La séquence EST (AI084125) est contiguë au site d'épissage interne de l'exon A. Nous n'avons pas encore déterminé avec précision le début des exons I, II et A puisque leurs séquences ont été déduites à partir de criblages de banques de cDNA (Figure 9A).

Quatre boîtes GC (GC 1 à GC4) ont été trouvées dans la région 5' (Figure 9B). Ces boîtes représentent des sites potentiels de liaison pour le facteur de transcription Sp1, mais seulement une boîte (GC3) correspond à une séquence consensus, c'est-à-dire GGGGCGGGG. De plus deux boîtes CCAAT (CB1 et CB2) ont été trouvées. Des analyses prédictives de séquences suggèrent que deux régions promotrices existent dans la région 5', c'est-à-dire avant le codon d'initiation (ATG). Deux sites potentiels d'initiation de la transcription ont été prédits aux positions 571 et 827. Le premier suit la séquence consensus de liaison à Sp1 et le second suit la boîte GC1 (respectivement entre les exons I & II, et les exons II & A). Afin de voir si ces deux régions sont fonctionnelles en tant que région promotrice, plusieurs constructions plasmidiques contenant un gène rapporteur (gène de la Chloramphénicol Acétyl Transférase; CAT) en aval des différentes régions promotrices potentielles ont été préparées. Des cellules COS ont été transfectées avec ces constructions plasmidiques. La figure 10 montre les résultats obtenus et qui correspondent au pourcentage d'augmentation de l'activité basale. L'activité maximale a été obtenue avec la construction plasmidique contenant 1114 paires de bases en amont du site d'initiation de la traduction, avec une augmentation de 236,7% de l'activité promotrice basale (promoteur minimal du gène de la thymidine kinase). La construction plasmidique contenant 642 paires de bases en amont de l'ATG a conduit à une augmentation de 115,6% alors que la construction plasmidique contenant uniquement la séquence entre l'exon I et l'exon II montrait une activité relativement faible avec une augmentation uniquement de 22,8% (figure 10). Ces résultats suggèrent l'existence d'une région promotrice entre les exons II et A.

### 3.2.3. Localisation chromosomique du gène ICBP90

La localisation chromosomique du gène ICBP90 a été réalisée par hybridation *in situ* de fluorescence (FISH). Le gène ICBP90 est localisé sur le chromosome 19p13.3 dans une région télomérique. Une recherche réalisée dans Genbank a montré qu'une région de 6Mb dans la bande chromosomique 19 p 13.3 d'une banque de cosmide spécifique du chromosome 19 (hybride homme / hamster 5HL2-B) contient 147 nucléotides codant pour les acides aminés 746 à 793 de ICBP90. Cette séquence a été localisée entre les marqueurs STS (sequence tagged site) D19S883 et D 19S325.

### 3.2.4 Expression de ICBP90 dans différentes lignées cellulaires

ICBP90 participe à la régulation de l'expression du gène TopIIα (Hopfner *et al*. (2000)). Comme TopIIα est exprimée de manière différentielle dans différentes tumeurs et lignées cellulaires, ICBP90 lui-même est susceptible d'avoir une régulation complexe en terme d'activité et d'expression génique.

Dans une première étape vers la compréhension des mécanismes régulant l'expression du gène ICBP90, l'ARNₘ d'ICBP90 a été analysé dans différentes lignées cellulaires. L'ARNₘ d'ICBP90 a été étudié dans la lignée cellulaire HL60 dérivée d'une leucémie promyélocytaire (ligne 1), de cellule Hela S3 (ligne 2), de cellules de leucémie lymphoblastique MOLT-4, de cellules Raji du lymphome de Burkitt (ligne 5), d'adénocarcinome colorectal SW 480 (ligne 6), de cellules A549 de carcinome du poumon (ligne 7) (figure 11A).

Deux bandes d'ARNₘ de 4,3 et 5,1 kb sont observées. Les quantités relatives d'ARNₘ dans les bandes varient selon le type cellulaire. L'histogramme de la figure 11A montre les taux d'ARNₘ dans les bandes de chacune des lignées cellulaires, exprimé en pourcentage de la quantité maximale observée de bandes d'ARNₘ de 5,1 kb dans les cellules HL-60 (ligne 1, figure 11A). Dans les cellules MOLT-4, seule la bande d'ARNₘ de 4,3 kb est observée, alors que dans les cellules de leucémies promyélocytaires la bande à 5,1 kb est prédominante. Dans les cellules Raji du lymphome de Burkitt, seule la bande à 5,1 kb est détectée. Approximativement, des quantités égales des deux types d'ARNₘ sont observées dans les autres lignées cellulaires, c'est-à-dire les cellules Hela, K562, A549, SW 480. Pour les cellules HL-60, néanmoins, l'ARNₘ de 5,1 kb est plus fortement exprimé que l'ARNₘ de 4,3 kb. D'autres analyses ont été entreprises sur les cellules Hela pour confirmer que les 2 transcrits proviennent de la transcription du gène ICBP90. Une sonde d'ADNc de 626 pb marquée à la digoxigénine localisée immédiatement en amont du signal poly A (c'est-à-dire l'exon F) et utilisée comme sonde pour des expériences de Northern Blotting, a produit les mêmes résultats, c'est-à-dire l'apparition de deux bandes d'ARNₘ de 4,3 kb et de 5,1 kb. Ce résultat confirme que les deux formes d'ARNₘ sont générées à partir d'un seul gène.

Les inventeurs ont également étudié l'expression de la protéine ICBP90 afin de déterminer si ces deux isoformes d'ARNₘ sont susceptibles de coder pour deux protéines différentes.

La figure 11B montre le profil d'expression de ICBP90 dans des extraits protéiques de cellules MOLT-4 et Hela. Alors qu'une seule bande de 97 kDa est observée dans les cellules MOLT-4, dans les cellules Hela, à côté de la bande de 97 kDa qui est doublée, plusieurs autres bandes d'un poids moléculaire inférieur sont observées. Ces résultats suggèrent que, dans les cellules MOLT-4, un ARNₘ code pour une forme unique d'ICBP90. A l'inverse, dans les cellules Hela, les deux ARNₘ sont susceptibles de conduire à la production de différents isoformes d'ICBP90.

### 3.3 Commentaires

Le gène ICBP90 s'étend sur environ 35,8 kb. Six exons traduits et deux exons non traduits, et de fait sept introns, ont été identifiés par les inventeurs. Les deux domaines en doigt de zinc de ICBP90 sont codés par le même exon (exon F), contrairement au gène du récepteur aux oestrogènes humains dans lequel chacun des doigts-de-zinc putatifs du domaine de liaison à l'ADN du récepteur sont codés séparément (Ponglikitmongkol *et al.* (1988)). Le domaine « ubiquitin-like » de ICBP90 est codé par les exons A et B alors que le domaine « leucine zipper » est codé par l'exon B. De manière intéressante, l'exon F seul est susceptible de coder pour une protéine fonctionnelle car elle code pour deux signaux de localisation nucléaire, les domaines zinc-finger et plusieurs sites putatifs de phosphorylation. Deux grands introns de 8,7 kb et de 19 kb ont été trouvés.

Le gène ICPB 90 a été localisé dans la région chromosomique 19p13.3. Plusieurs autres gènes ont été localisés dans cette région, par exemple le Nuclear Factor I/C (également un facteur de transcription liant CCAAT, (Qian *et al*. (1995)). De manière intéressante, une translocation atypique t (7 ; 19) dans la leucémie myélomonocytaire aiguë, impliquant un site fragile au locus 19p13.3 a été décrite (Sherer *et al*. (1991)). Egalement, il a été suggéré que les gènes impliqués dans le développement des carcinomes pancréatiques sont localisés en 19p13.3 et 19q13.1-13.2 (Hoglund *et al*. (1998)). Des réarrangements des bandes 14q32.3 et 19p13.3 d'une délétion préférentielle du bras court du chromosome 1 constituent des altérations chromosomiques non aléatoires dans le myélome multiple et la leucémie des cellules du plasma (Taniwaki *et al*. (1996)). D'autres gènes ont été localisés dans cette région ; ils incluent un gène impliqué dans l'adénocarcinome du syndrome Peutz-Jeghers (Gruba *et al*. (1998)). Egalement, il a été suggéré que le gène suppresseur de tumeur putatif pour l'adénome malin est localisé en D19S216 au niveau de la bande chromosomique 19p 13.3 qui joue un rôle important dans la tumorigenèse de l'adénome malin (Lee *et al*. (1998)).

L'analyse de la séquence de la région 5' du gène ICBP90 a révélé l'existence de plusieurs exons non-traduits avec une région promotrice entre les exons II et A et probablement un second promoteur plus faible localisé entre les exons I et II. La région promotrice entre les exons II et A est un promoteur sans séquence TATA, suggérant que le gène ICBP90 peut être un gène de ménage, au moins lorsque ce promoteur est impliqué. En ce sens, il ressemble fortement aux régions promotrices des gènes ATFα (Goetz *et al*., 1996), CRE-BP1 / ATF 2 (Nagase *et al*., 1990) et TopIIα, (Hochhauser *et al*., 1992), qui ne contiennent pas de boîtes TATA canoniques mais plusieurs sites de liaison de SP-1.

Les boîtes GC et/ou CCAAT sont susceptibles d'être impliquées dans la régulation de l'expression du gène ICBP90 via les facteurs de transcription SP-1 et les protéines de liaison à CCAAT. De plus, étant donné que la protéine ICBP90 est une protéine de liaison à CCAAT, ICBP90 est également susceptible de réguler sa propre expression.

Une banque de données de facteurs de transcription a été criblée à l'aide du programme d'ordinateur Mat Inspector du Baylor College of Medicine, et de nombreux sites de liaison de facteurs de transcription ont été identifiés dans la séquence précédant le codon ATG (figure 9B). Parmi ces sites de liaison aux facteurs de transcription, il est intéressant de noter des sites de liaison du facteur de transcription AP-2 régulé au cours du développement et qui contrôle l'expression de gène tel DR-nm 23 (Martinez *et al*. (1997)), les sites de liaison de la protéine myéloide « zinc-finger » MZF 1 qui est impliquée dans la régulation de l'hématopoïèse (Hromas *et al*. (1996)).

L'analyse de Northern Blotting a démontré qu'il existe deux populations d'ARNₘ de 4,3 kb et de 5,1 kb. De manière intéressante, chaque population présente une spécificité cellulaire. Par exemple, les cellules lymphoblastiques MOLT-4 expriment seulement l'ARNₘ de 4,3 kb, alors que dans les cellules Raji du lymphome de Burkitt (lymphocytes B matures), seul le transcrit de 5,1 kb est observé. Les cellules HL-60 expriment davantage d'ARNₘ de 5,1 kb que d'ARNₘ de 4,3 kb. Les cellules HL-60 et les cellules Raji du lymphome de Burkitt sont davantage différenciées que les cellules MOLT-4 suggérant que les taux d'expression du transcrit de 5,1 kb par rapport à celui de 4,3 kb peut être directement corrélé avec l'état de différenciation des cellules.

De manière intéressante, une étiquette de séquence exprimée (EST, Expressed Sequence Tag) correspondant à la séquence 5' de l'exon A a été identifiée à partir d'oligodendrogliome anaplastique (numéro d'accession Genbank N° AI 084 125) alors qu'une EST correspondant à l'inclusion de l'exon II a été isolée à partir d'un mélange de tumeurs de cellules germinales (numéro d'accession Genbank N° AI 968 662). Les résultats des inventeurs suggèrent donc que la régulation des transcrits ICBP90 est comparable avec ce qui se passe pour le récepteur aux oestrogènes. En fait, six transcrits différents codant une protéine commune, mais différant dans la région 5' non traduite du fait d'un épissage alternatif des exons amonts, ont été reportés (Flouriot *et al*., 1998 et Grandien, 1996).

L'analyse en Western Blotting montre une bande majoritaire à 97 kDa dans les cellules MOLT-4 alors que plusieurs bandes sont observées dans les cellules Hela (Figure 11B). Ces données sont en accord avec l'existence de plusieurs ARNₘ ICBP90 et/ou d'isoformes de la protéine ICBP90 dont le taux d'expression peut être contrôlé de manière cellule-spécifique.

Deux isoformes protéiques pour le récepteur aux oestrogènes ont été décrits (Griffin *et al*., 1999) qui diffèrent l'un de l'autre par les 41 amino-acides N-terminaux. La double-bande de 97 kDa observée à partir des cellules Hela (Figure 11B) est donc susceptible de représenter deux isoformes différant par leur extrémité N-terminale. Pour ce faire, l'exon A codant pour 47 amino-acides est épissé en dehors du cadre de lecture, et conséquemment, la région protéique codante commence avec l'exon B. Néanmoins, il est également possible qu'il existe d'autres exons susceptibles d'être transcrits dans d'autres tissus.

Egalement, l'intron de 8,7 kb (c'est-à-dire entre l'exon D et E) est susceptible de contenir une région promotrice qui peut conduire à des isoformes d'ICBP90 de poids moléculaires inférieurs à ceux observés dans les cellules Hela en prolifération (Figure 11B). De manière intéressante, la spécificité tissulaire des différents ARNₘ du récepteur aux oestrogènes est déterminée par différents promoteurs dont l'activité apparaît être altérée dans les lignées cellulaires du cancer du sein (Flouriot *et al*., 1998).

L'ensemble de ces résultats suggère que le gène ICBP90 et la protéine ICBP90 présentent des caractéristiques communes avec les membres de la famille du récepteur à l'acide rétinoïque, aux stéroïdes, aux hormones thyroïdiennes en ce qui concerne les structures génique et protéique.

En effet, les inventeurs ont démontré expérimentalement, en utilisant la technique du double hybride, l'existence d'interactions entre la protéine ICBP90 et TIP60 (Tat Interactive Protein, 60 kDa). La protéine TIP60 a été très récemment décrite comme étant un coactivateur du récepteur nucléaire, notamment le récepteur pour les androgènes (Brady ME *et al*., 1999).

De ce fait, ICBP90 est susceptible de jouer le rôle d'un récepteur nucléaire sur lequel se lie un ligand endogène. Il est donc également dans la portée de la présente invention d'utiliser le polypeptide ICBP90 de l'invention pour isoler, cribler, identifier le ligand endogène. Il est également dans la portée de l'invention d'utiliser le polypeptide ICBP90 de l'invention pour isoler, cribler, identifier des molécules naturelles ou de synthèse, biologique ou chimique, agoniste ou antagoniste de ce ligand naturel.

### REFERENCES

Austin et al. (1993), Biochim. Biophys. Acta, 1172, 283-291.
Banerji, J. et al. (1981), Cell, 27 : 299-308.
Barany, F. (1991), Proc. Natl. Acad. Sci. USA, 88, 189-193.
Boritzki, T. J. et al. (1988), Biochem. Pharmacol., 37, 4063-4068.
Brady, M. E. et al. (1999), J. Biol. Chem., 274 : 17599-17604.
Brandt, T.L. et al. (1997), J. Biol. Chem., 272, 6278-6284.
Brou, C. et al. (1993), EMBO J., 12, 489-499.
Buckholz, R. G. (1993), Curr. Op. Biotechnology, 4, 538-542.
Burg, J.L. et al. (1996), Mol. and Cell. Probes, 10, 257-271.
Chu, B.C.F. et al. (1986), Nucleic Acids Res., 14, 5591-5603.
Chung, T.D.Y. et al. (1989), Proc. Natl. Acad. Sci. USA, 86, 9431-9435.
Darzynkiewicz et al. (1994), Methods in Cells Biology, 41, 421-435.
Deffie, A.M. et al. (1989), Cancer Res., 49, 58-62.
De Vries, L. et al. (1995), Proc. Natl. Acad. Sci. USA, 92, 11916-11920.
Devys et al. (1993), Nature Genet., 4, 335-340.
Drake, F. H. et al., Biochemistry, 28, 8154-8160.
Duck, P. et al. (1990), Biotechniques, 9, 142-147.
Edwards, C.P. and Aruffo, A. (1993), Curr. Op. Biotechnology, 4, 558-563.
Erlich, H.A. (1989), New York : Stockton Press.
Flouriot et al. (1998), Mol. Endrocrinol., 12 : 1239-254.
Fry, A.M. et al. (1991), Cancer Res., 51, 6592-6595.
Furth et al. (1992), Anal. Biochem., 205 : 365.
Gaub, M.P. et al. (1998), J. Histochem Cytochem., 46, 1103-1111.
Goetz, J. et al. (1996), J. Biol. Chem., 271 : 29589-29598.
Goswami, P.C. et al. (1996), Mol. Cell. Biol., 16, 1500-1508.
Grandien (1996), Mol. Cell. Endrocrinol., 116 : 207-212).
Griffin et al. (1999), Mol. Endocrinol. 13 : 1571-1587.
Gruba et al. (1998), Cancer Res., 58 : 5267-5270.
Guatelli, J.C. et al. (1990), Proc. Natl. Acad. Sci. USA, 87, 1874-1878.
Guinee, D.G. et al. (1996), Cancer, 78, 729-735.
Haddad et al. (1988), Human Genet., 103 : 619-625.
Heck, M.M. et al. (1988), Proc. Natl. Acad. Sci. USA, 85, 1086-1090.
Herzog, C.E. and Zwelling, L. A. (1997), Biochem. Biophys. Res. Commun., 232, 608-612.
Hochhauser, D. et al. (1992), J. Biol. Chem., 267, 18961 - 18965.
Hoglund et al. (1998), Genes Chromosomes Cancer, 21 : 8-16.
Hopfner et al. (2000), Cancer Res., 60 : 121-128.
Hromas et al. (1996), Curr. Top. Microbiol. Chem., 211 : 159-164.
Innis, M.A. *et al*. (1990), Academic Press.
Inouye, C. et al. (1994), DNA Cell Biol., 13, 731-742.
Isaacs, R.J. et al., Biochim. Biophys. Acta, 1400, 121-137.
Isaacs, R.J. et al. (1996), J. Biol. Chem., 271, 16741-16747.
Jenkins, J.R. et al. (1992), Nucleic Acids Res., 20, 5587-5592. Kassel, O. et al. (1998), Mol. Pharmacol., 54, 1073-1079.
Kennerdell, J.R. and Carthew, R.W. (1998), Cell, 95, 1017-1026.
Kievitis, T. et al. (1991), J. Virol. Methods, 35, 273-286.
Kohler, G. et al. (1975), Nature, 256 (5517), 495-497.
Kubo, T. et al. (1995), Cancer Res., 55, 3860-3864.
Kwoh, D.Y. et al. (1989), Proc. Natl. Acad. Sci. USA, 86, 1173-1177.
Landegren, U. et al. (1988), Science, 241, 1077-1080.
Lavie, J. et al. (1999), J. Biol. Chem., 274, 2308-2314.
Lee et al. (1998), Cancer Res., 58 : 1140-1143.
Lim, K. et al. (1998), Biochem. Mol. Biol. Int., 46, 35-42.
Lizardi, P.M. et al. (1988), Bio/technology, 6, 1197-1202.
Luckow, V.A. (1993), Curr. Op. Biotechnology, 4, 564-572.
Martinez et al. (1997), Cancer Res., 57 : 1180-1187.
Matthews, J.A. et al. (1988), Anal. Biochem., 169 : 1-25.
Miele, E.A. et al. (1983), J. Mol. Biol., 171 : 281-295.
Nagase et al. (1990), J. Biol. Chem., 265 : 17300-17306.
Nitiss, J.L. (1998), Biochim. Biophys. Acta, 1400, 63-81.
Olins, P.O. and Lee, S.C. (1993), Curr. Op. Biotechnology, 4, 520-525.
Pommier, Y. et al. (1994), Cancer Invest., 12, 530-542.
Ponglikitmongkol et al. (1988), EMBO J. 7 : 3385-3388.
Rio, M.C. et al. (1987), Proc. Natl. Acad. Sci. USA, 84, 9243-9247.
Qian et al. (1995), Genomics, 28 : 66-73.
Reese et al. (1996), Large Scale Sequencing Specific Neural Networks for Promoter and Splice Recognition. Biocomputing : Proceedings of the 1996 Pacific Symposium. Edited by Lawrence Hunter and Terri E. World Scientific Singapore, 1996, January 27, 1996.
Rochette-Egly, C. et al. (1997), Cell, 90, 97-107.
Roskell, D.E. et Biddolph, S.C. (1999), Eur. J. Med. Res. 26, 105-106.
Rolfs, A. *et al.* (1991), Berlin : Springer-Verlag.
Sambrook, J. et al. (1989), Molecular Cloning: A Laboratory Manual, Sec. Ed. Cold Spring Harbor Lab., Cold Spring Harbor, New York.
Sandri, M.I. et al. (1996), Nucleic Acids Res., 24, 4464-4470.
Segev, D. (1992), Kessler C. Springer Verlag, Berlin, New-York, 197-205.
Sherer et al. (1991), Cancer Genet. Cytogenet., 57 : 169-173.
Stone, B.B. et al. (1996). Mol. and Cell. Probes, 10 : 359-370.
Tang et al. (1992), Nature, 356 : 152.
Taniwaki et al. (1996), Leuk. Lymphoma, 21 : 25-30.
Tsai-plugfelder, M. et al. (1988), Proc. Natl. Acad. Sci. USA, 85, 7177-7181.
Walker, G.T. et al. (1992), Nucleic Acids Res., 20 : 1691-1696.
Wang, J.C. (1996), Ann. Rev. Biochem., 65, 635-692.
Wang, M.M. and Reed, R.R. (1993), Nature (London), 364, 121-126.
Yamazaki et al. (1996), Acta Oncol., 35, 417-423.

## Revendications

1. Polypeptide isolé dénommé ICBP90 (inverted CCAAT box binding protein 90) de séquence d'acides aminés SEQ ID N°2.

2. Polypeptide isolé **caractérisé en ce qu'**il comprend un polypeptide choisi parmi un polypeptide de séquence SEQ ID N°2, SEQ ID N°4, SEQ ID N°6, SEQ ID N°8 ou le fragment C-terminal à partir du résidu D263 de SEQ ID N°2.

3. Polypeptide selon la revendication 1 ou 2 **caractérisé en ce que** la séquence d'ADN sur laquelle se lie ledit polypeptide est une boîte CCAAT, de préférence une boîte CCAAT inversée (Inverted CCAAT Box : ICB).

4. Polynucléotide isolé **caractérisé en ce qu'**il code pour un polypeptide selon la revendication 1.

5. Polynucléotide selon la revendication 4 de séquence SEQ ID N°1.

6. Polynucléotide isolé **caractérisé en ce qu'**il comprend un polynucléotide choisi parmi un polynucléotide de séquence SEQ ID N°1, SEQ ID N°3, SEQ ID N°5, SEQ ID N°7 ou codant pour le fragment C-terminal à parti du résidu D263 de SEQ ID N°2.

7. Vecteur recombinant de clonage d'un polynucléotide selon l'une des revendications 4 à 6 et/ou d'expression d'un polypeptide selon l'une des revendications 1 à 3 **caractérisé en ce qu'**il contient un polynucléotide selon l'une quelconque des revendications 4 à 6.

8. Vecteur selon la revendication 7 **caractérisé en ce qu'**il comporte les éléments permettant l'expression éventuellement la sécrétion dudit polypeptide dans une cellule hôte.

9. Vecteur selon l'une quelconque des revendications 7 à 8 **caractérisé en ce que** les éléments permettant l'expression dudit polypeptide sont choisis parmi :
a) le polynucléotide isolé de séquence SED ID N°12 ;
b) un polynucléotide dont la séquence est complémentaire de la séquence du polynucléotide défini en a) ;
c) un polynucléotide dont la séquence comporte au moins 80% d'identité avec un polynucléotide défini en a) ou en b) ;
d) un polynucléotide hybridant dans des conditions de forte stringence avec une séquence du polynucléotide défini en a), b) ou c).

10. Cellule hôte, **caractérisée en ce qu'**elle comprend un vecteur selon l'une des revendications 7, 8 et 9.

11. Procédé de préparation d'un polypeptide recombinant **caractérisé en ce que** l'on cultive une cellule hôte selon la revendication 10 dans des conditions permettant l'expression et éventuellement la sécrétion dudit polypeptide recombinant et que l'on récupère ledit polypeptide recombinant.

12. Polypeptide recombinant susceptible d'être obtenu par un procédé selon la revendication 11.

13. Anticorps monoclonal ou polyclonal et ses fragments **caractérisé en ce qu'**il lie spécifiquement un polypeptide choisi parmi : SEQ ID NO :2, 4, 6 ou 8.

14. Procédé de détection et/ou de dosage d'un polypeptide selon l'une des revendications 1 à 3, dans un échantillon biologique, **caractérisé en ce qu'**il comprend les étapes suivantes :
a) mise en contact de l'échantillon biologique avec un anticorps selon la revendication 13 ;
b) mise en évidence du complexe antigène-anticorps formé.

15. Nécessaire pour la mise en oeuvre d'un procédé selon la revendication 14 dans un échantillon biologique par réaction immunologique, **caractérisé en ce qu'**il comprend les éléments suivants :
a) un anticorps monoclonal ou polyclonal selon la revendication 13 ;
b) le cas échéant, les réactifs pour la constitution du milieu propice à la réaction immunologique ;
c) les réactifs permettant la détection du complexe antigène-anticorps produit par la réaction immunologique.

16. Procédé selon la revendication 14 pour le diagnostic de prolifération cellulaire.

17. Procédé de criblage de ligand susceptible d'affecter l'activité transcriptionnelle d'un gène dont le promoteur comporte des boîtes CCAAT et/ou CCAAT inversées (ICB) susceptibles de lier un polypeptide selon les revendications 1 à 3 et qui comporte les étapes suivantes :
a) mise en contact dudit polypeptide et d'un ou plusieurs ligand(s) potentiel(s) en présence de réactifs nécessaires à la mise en oeuvre d'une réaction de transcription;
b) détection et/ou mesure de l'activité transcriptionnelle.

18. Procédé de criblage de ligand susceptible d'affecter la fonction « récepteur nucléaire » du polypeptide selon les revendications 1 à 3 et qui comporte les étapes suivantes :
a) mise en contact dudit polypeptide et d'un ou plusieurs ligands potentiels en présence de réactifs nécessaires ;
b) détection et/ou mesure de l'activité transcriptionnelle d'un gène dont le promoteur comporte des boîtes CCAAT et/ou CCAAT inversées (ICB) susceptibles de lier ledit polypeptide.

19. Nécessaire pour la mise en oeuvre d'un procédé selon les revendications 17 et 18 dans un échantillon biologique **caractérisé en ce qu'**il comprend les éléments suivants :
a) un polypeptide selon les revendications 1 à 3 ;
b) un ligand ;
c) les réactifs nécessaires à la mise en oeuvre d'une réaction de transcription.

20. Composé **caractérise en ce qu'**il est choisi parmi :
a) un polypeptide selon l'une des revendications 1 à 3 ;
b) un polynucléotide selon l'une des revendications 4 à 6 ;
c) un vecteur selon l'une des revendications 7 à 9 ;
d) une cellule selon la revendication 10 ;
e) un anticorps selon la revendication 13 ;
à titre de médicament.

21. Composé selon la revendication 20 à titre de médicament destiné à la prévention et/ou au traitement du cancer.

22. Utilisation d'un composé selon les revendications 20 et 21 pour la préparation d'un médicament destiné à moduler, à augmenter ou à diminuer la prolifération cellulaire.

23. Composition pharmaceutique pour le traitement préventif et curatif du cancer **caractérisé en ce qu'**elle contient une quantité thérapeutiquement efficace d'un composé selon l'une des revendications 20 et 21 et un véhicule pharmaceutiquement acceptable.

24. Composition pharmaceutique **caractérisée en ce qu'**elle comprend un anticorps selon la revendication 13 en tant qu'agent de ciblage conjugué à au moins un agent sélectionné parmi le groupe des agents antiprolifératifs, antinéoplastiques ou cytotoxiques.

25. Produit comprenant au moins un composé selon les revendications 20 et 21 et au moins un autre agent anticancéreux comme produit de combinaison pour une utilisation simultanée, séparée ou étalée dans le temps en thérapie anti-cancéreuse.

26. Composition pour la détection, la localisation et l'imagerie des cancers, comprenant un anticorps selon la revendication 13, l'anticorps est marqué directement ou indirectement avec un marqueur générateur de signal sélectionné parmi les isotropes radioactifs et les entités non isotopiques.

## Claims

1. Isolated polypeptide named ICBP90 (inverted CCAAT box binding protein 90) of amino acid sequence SEQ ID No. 2.

2. Isolated polypeptide, **characterised in that** it comprises a polypeptide selected from a polypeptide of sequence SEQ ID No. 2, SEQ ID No. 4, SEQ ID No. 6, SEQ ID No. 8 or the C-terminal fragment starting from residue D263 of SEQ ID No. 2.

3. Polypeptide according to claim 1 or 2, **characterised in that** the DNA sequence to which said polypeptide binds is a CCAAT box, preferably an inverted CCAAT box (ICB).

4. Isolated polynucleotide, **characterised in that** it codes for a polypeptide according to claim 1.

5. Polynucleotide according to claim 4 of sequence SEQ ID No. 1.

6. Isolated polynucleotide, **characterised in that** it comprises a polynucleotide selected from a polynucleotide of sequence SEQ ID No. 1, SEQ ID No. 3, SEQ ID No. 5, SEQ ID No. 7 or coding for the C-terminal fragment starting from residue D263 of SEQ ID No. 2.

7. Recombinant vector for cloning a polynucleotide according to one of claims 4 to 6 and/or expression of a polypeptide according to one of claims 1 to 3, **characterised in that** it contains a polynucleotide according to any one of claims 4 to 6.

8. Vector according to claim 7, **characterised in that** it includes the elements allowing the expression and optionally secretion of said polypeptide in a host cell.

9. Vector according to any one of claims 7 to 8, **characterised in that** the elements allowing the expression of said polypeptide are selected from:
a) the isolated polynucleotide of sequence SEQ ID No. 12;
b) a polynucleotide whose sequence is complementary to the sequence of the polynucleotide defined in a);
c) a polynucleotide whose sequence has at least 80% identity with a polynucleotide defined in a) or in b);
d) a polynucleotide hybridising under high-stringency conditions with a sequence of the polynucleotide defined in a), b) or c).

10. Host cell, **characterised in that** it comprises a vector according to one of claims 7, 8 and 9.

11. Method of preparing a recombinant polypeptide, **characterised in that** a host cell according to claim 10 is cultured under conditions allowing the expression and optionally secretion of said recombinant polypeptide and **in that** said recombinant polypeptide is recovered.

12. Recombinant polypeptide capable of being obtained by a method according to claim 11.

13. Monoclonal or polyclonal antibody and fragments thereof, **characterised in that** it specifically binds a polypeptide selected from: SEQ ID No.: 2, 4, 6 or 8.

14. Method of detecting and/or assaying a polypeptide according to one of claims 1 to 3, in a biological sample, **characterised in that** it comprises the following steps:
a) placing the biological sample in contact with an antibody according to claim 13;
b) determining the antigen-antibody complex formed.

15. Kit for carrying out a method according to claim 14 in a biological sample by means of immunological reaction, **characterised in that** it comprises the following elements:
a) a monoclonal or polyclonal antibody according to claim 13;
b) where appropriate, reagents for forming the medium favourable to the immunological reaction;
c) reagents allowing detection of the antigen-antibody complex produced by the immunological reaction.

16. Method according to claim 14 for diagnosing cell proliferation.

17. Method of screening for a ligand capable of affecting the transcriptional activity of a gene whose promoter has CCAAT boxes and/or inverted CCAAT boxes (ICB) which are capable of binding a polypeptide according to claims 1 to 3, and which includes the following steps:
a) placing said polypeptide and one or more potential ligand(s) in contact in the presence of reagents necessary for carrying out a transcription reaction;
b) detection and/or measurement of the transcriptional activity.

18. Method of screening for a ligand capable of affecting the "nuclear receptor" function of the polypeptide according to claims 1 to 3, and which includes the following steps:
a) placing said polypeptide and one or more potential ligands in contact in the presence of necessary reagents;
b) detection and/or measurement of the transcriptional activity of a gene whose promoter has CCAAT boxes and/or inverted CCAAT boxes (ICB) which are capable of binding said polypeptide.

19. Kit for carrying out a method according to claims 17 and 18 in a biological sample, **characterised in that** it comprises the following elements:
a) a polypeptide according to claims 1 to 3;
b) a ligand;
c) reagents necessary for carrying out a transcription reaction.

20. Compound, **characterised in that** it is selected from:
a) a polypeptide according to one of claims 1 to 3;
b) a polynucleotide according to one of claims 4 to 6;
c) a vector according to one of claims 7 to 9;
d) a cell according to claim 10;
e) an antibody according to claim 13;
as a medicament.

21. Compound according to claim 20 as a medicament intended for the prevention and/or treatment of cancer.

22. Use of a compound according to claims 20 and 21 in preparation of a medicament intended to modulate, increase or reduce cell proliferation.

23. Pharmaceutical composition for the preventative and curative treatment of cancer, **characterised in that** it contains a therapeutically efficacious amount of a compound according to one of claims 20 and 21 and a pharmaceutically acceptable carrier.

24. Pharmaceutical composition, **characterised in that** it comprises an antibody according to claim 13 as a targeting agent conjugated with at least one agent selected from the group of anti-proliferative, anti-neoplastic or cytotoxic agents.

25. Product comprising at least one compound according to claims 20 and 21 and at least one other anti-cancer agent as a combination product for simultaneous, separate or sequential use in anti-cancer therapy.

26. Composition for detecting, localising and imaging cancers, comprising an antibody according to claim 13, which antibody is labelled directly or indirectly with a signal-generating label selected from radioactive isotopes and non-isotopic entities.

## Patentansprüche

1. Isoliertes Polypeptid, das als ICBP90 (inverted CCAAT box binding protein 90) bezeichnet wird, mit der Aminosäuresequenz SEQ ID No. 2.

2. Isoliertes Polypeptid, **dadurch gekennzeichnet, dass** es ein Polypeptid umfasst, das aus einem Polypeptid der SEQ ID No. 2, SEQ ID No. 4, SEQ ID No. 6, SEQ ID No. 8 oder dem C-terminalen Fragment ab dem Rest D263 der SEQ ID No. 2 ausgewählt wird.

3. Polypeptid gemäß Anspruch 1 oder 2, **dadurch gekennzeichnet, dass** die DNA-Sequenz, an die das Polypeptid bindet, eine CCAAT-Box, vorzugsweise eine invertierte CCAAT-Box (engl. Inverted CCAAT Box: ICB) ist.

4. Isoliertes Polynukleotid, **dadurch gekennzeichnet, dass** es ein Polypeptid gemäß Anspruch 1 kodiert.

5. Polynukleotid gemäß Anspruch 4 der Sequenz SEQ ID No. 1.

6. Isoliertes Polynukleotid, **dadurch gekennzeichnet, dass** es ein Polynukleotid umfasst, das aus einem Polynukleotid der Sequenz SEQ ID No. 1, SEQ ID No. 3, SEQ ID No. 5, SEQ ID No. 7 ausgewählt wird oder das C-terminale Fragment ab dem Rest D263 der SEQ ID No. 2 kodiert.

7. Rekombinanter Vektor zum Klonieren eines Polynukleotids gemäß einem der Ansprüche 4 bis 6 und/oder zum Exprimieren eines Polypeptids gemäß einem der Ansprüche 1 bis 3, **dadurch gekennzeichnet, dass** er ein Polynukleotid gemäß einem der Ansprüche 4 bis 6 umfasst.

8. Vektor gemäß Anspruch 7, **dadurch gekennzeichnet, dass** er die Elemente umfasst, die die Expression in und gegebenenfalls die Sekretion des Polypeptids aus einer Wirtszelle ermöglicht.

9. Vektor gemäß irgendeinem der Ansprüche 7 bis 8,
**dadurch gekennzeichnet, dass** die Elemente, die die Expression des Polypeptids ermöglichen, ausgewählt werden aus:
a) dem isolierten Polynukleotid der Sequenz SEQ ID No. 12,
b) einem Polynukleotid, dessen Sequenz komplementär zu der Sequenz des unter a) definierten Polynukleotids ist,
c) einem Polynukleotid, dessen Sequenz mindestens 80 % Identität mit einem unter a) oder unter b) definierten Polynukleotid hat,
d) einem Polynukleotid, das unter Bedingungen einer hohen Stringenz mit einer Sequenz des unter a), b) oder c) definierten Polynukleotids hybridisiert.

10. Wirtszelle, **dadurch gekennzeichnet, dass** sie einen Vektor gemäß einem der Ansprüche 7, 8 und 9 umfasst.

11. Verfahren zum Herstellen eines rekombinanten Polypeptids, **dadurch gekennzeichnet, dass** eine Wirtszelle gemäß Anspruch 10 unter Bedingungen kultiviert wird, die die Expression und gegebenenfalls die Sekretion des rekombinanten Polypeptids ermöglichen, und dass das rekombinante Polypeptid gesammelt wird.

12. Rekombinantes Polypeptid, das mittels eines Verfahrens gemäß Anspruch 11 erhalten werden kann.

13. Monoklonaler oder polyklonaler Antikörper und dessen Fragmente, **dadurch gekennzeichnet, dass** er insbesondere an ein Polypeptid bindet, das aus der SEQ ID No. 2, 4, 6 oder 8 ausgewählt wird.

14. Verfahren zum Nachweis und/oder der Bestimmung der Menge eines Polypeptids gemäß einem der Ansprüche 1 bis 3 in einer biologischen Probe, **dadurch gekennzeichnet, dass** es die folgenden Schritte umfasst:
a) In-Kontakt-Bringen der biologischen Probe mit einem Antikörper gemäß Anspruch 13,
b) Nachweis des gebildeten Antigen-Antikörper-Komplexes.

15. Kit zum Durchführen eines Verfahrens gemäß Anspruch 14 mit einer biologischen Probe mittels Immunreaktion, **dadurch gekennzeichnet, dass** es die folgenden Elemente umfasst:
a) einen monoklonalen oder polyklonalen Antikörper gemäß Anspruch 13,
b) gegebenenfalls Reagenzien zum Bilden der für die Immunreaktion günstigen Bedingungen,
c) Reagenzien, die den Nachweis des durch die Immunreaktion erzeugten Antigen-Antikörper-Komplexes ermöglichen.

16. Verfahren gemäß Anspruch 14 für die Diagnose einer Zellproliferation.

17. Verfahren zum Screening eines Liganden, der geeignet ist, die Transkriptionsaktivität eines Gens zu beeinflussen, dessen Promotor CCAAT-Boxen und/oder invertierte CCAAT-Boxen (ICB) umfasst, die geeignet sind, an ein Polypeptid gemäß den Ansprüchen 1 bis 3 zu binden, und das die folgenden Schritte umfasst:
a) In-Kontakt-Bringen des Polypeptids mit einem oder mehreren potentiellen Liganden in Anwesenheit von Reagenzien, die für das Durchführen einer Transkriptionsreaktion erforderlich sind,
b) Nachweisen und/oder Messen der Transkriptionsaktivität.

18. Verfahren zum Screening eines Liganden, der geeignet ist, die "Kernrezeptor"-Funktion des Polypeptids gemäß den Ansprüchen 1 bis 3 zu beeinflussen, und das die folgenden Schritte umfasst:
a) In-Kontakt-Bringen des Polypeptids mit einem oder mehreren potentiellen Liganden in Anwesenheit der erforderlichen Reagenzien,
b) Nachweisen und/oder Messen der Transkriptionsaktivität eines Gens, dessen Promotor CCAAT-Boxen und/oder invertierte CCAAT-Boxen (ICB) umfasst, die geeignet sind, an das Polypeptid zu binden.

19. Kit zum Durchführen eines Verfahrens gemäß den Ansprüchen 17 und 18 mit einer biologischen Probe, **dadurch gekennzeichnet, dass** es die folgenden Elemente umfasst:
a) ein Polypeptid gemäß den Ansprüchen 1 bis 3,
b) einen Liganden,
c) die für das Durchführen einer Transkriptionsreaktion notwendigen Reagenzien.

20. Zusammensetzung, **dadurch gekennzeichnet, dass** sie aus
a) einem Polypeptid gemäß einem der Ansprüche 1 bis 3,
b) einem Polynukleotid gemäß einem der Ansprüche 4 bis 6,
c) einem Vektor gemäß einem der Ansprüche 7 bis 9,
d) einer Zelle gemäß Anspruch 10,
e) einem Antikörper gemäß Anspruch 13
ausgewählt wird, als Medikament.

21. Zusammensetzung gemäß Anspruch 20 als Medikament, das zur Vorbeugung gegen und/oder zur Behandlung von Krebs bestimmt ist.

22. Verwendung einer Zusammensetzung gemäß den Ansprüchen 20 und 21 zur Herstellung eines Medikaments, das bestimmt ist, um die Zellproliferation zu modulieren, zu erhöhen oder zu verringern.

23. Pharmazeutische Zusammensetzung für die präventive und kurative Behandlung von Krebs, **dadurch gekennzeichnet, dass** sie eine therapeutisch wirksame Menge einer Verbindung gemäß einem der Ansprüche 20 und 21 und einen pharmazeutisch annehmbaren Träger umfasst.

24. Pharmazeutische Zusammensetzung, **dadurch gekennzeichnet, dass** sie einen Antikörper gemäß Anspruch 13 als gezielt wirkenden Stoff umfasst, der mit mindestens einem Wirkstoff konjugiert ist, der aus der Gruppe der antiproliferativen, antineoplastischen oder zytotoxischen Wirkstoffe ausgewählt wird.

25. Produkt, das mindestens eine Verbindung gemäß den Ansprüchen 20 und 21 und mindestens einen weiteren krebshemmenden Stoff umfasst, als Kombinationsprodukt für eine gleichzeitige, getrennte oder über den Zeitraum der Antikrebs-Therapie verteilte Verwendung.

26. Zusammensetzung zum Nachweisen, zum Lokalisieren und für die Bildgebung von Krebs, umfassend einen Antikörper gemäß Anspruch 13, wobei der Antikörper direkt oder indirekt mit einem signalerzeugenden Marker markiert wird, der aus Radioisotopen und nicht-isotopen Entitäten ausgewählt wird.


### LISTE DE SEQUENCES

<110> ADEREGEM
<120> Polypeptide ICBP90 et ses fragments et polynucléotides codant lesdits polypeptides et applications au diagnostic et au traitement du cancer
<130> d18243
<140>
   <141>
<160> 12
<170> PatentIn Ver. 2.1
<210> 1
   <211> 2382
   <212> ADN
   <213> Homo sapiens
<220>
   <221> CDS
   <222> (1)..(2382)
<400> 1
   atg tgg atc cag gtt cgg acc atg gat ggg agg cag acc cac acg gtg      48
   Met Trp Ile Gln Val Arg Thr Met Asp Gly Arg Gln Thr His Thr Val
    1               5                  10                  15
   gac tcg ctg tcc agg ctg acc aag gtg gag gag ctg agg cgg aag atc      96
   Asp Ser Leu Ser Arg Leu Thr Lys Val Glu Glu Leu Arg Arg Lys Ile
               20                  25                  30
   cag gag ctg ttc cac gtg gag cca ggc ctg cag agg ctg ttc tac agg     144
   Gln Glu Leu Phe His Val Glu Pro Gly Leu Gln Arg Leu Phe Tyr Arg
           35                  40                  45
   ggc aaa cag atg gag gac ggc cat acc ctc ttc gac tac gag gtc cgc     192
   Gly Lys Gln Met Glu Asp Gly His Thr Leu Phe Asp Tyr Glu Val Arg
       50                  55                  60
   ctg aat gac acc atc cag ctc ctg gtc cgc cag agc ctc gtg ctc ccc     240
   Leu Asn Asp Thr Ile Gln Leu Leu Val Arg Gln Ser Leu Val Leu Pro
   65                  70                  75                  80
   cac agc acc aag gag cgg gac tcc gag ctc tcc gac acc gac tcc ggc     288
   His Ser Thr Lys Glu Arg Asp Ser Glu Leu Ser Asp Thr Asp Ser Gly
                   85                  90                  95
   tgc tgc ctg ggc cag agt gag tca gac aag tcc tcc acc cac ggt gag     336
   Cys Cys Leu Gly Gln Ser Glu Ser Asp Lys Ser Ser Thr His Gly Glu
               100                 105                 110
   gcg gcc gcc gag act gac agc agg cca gcc gat gag gac atg tgg gat     384
   Ala Ala Ala Glu Thr Asp Ser Arg Pro Ala Asp Glu Asp Met Trp Asp
           115                 120                 125
   gag acg gaa ttg ggg ctg tac aag gtc aat gag tac gtc gat gct cgg     432
   Glu Thr Glu Leu Gly Leu Tyr Lys Val Asn Glu Tyr Val Asp Ala Arg
       130                 135                 140
   gac acg aac atg ggg gcg tgg ttt gag gcg cag gtg gtc agg gtg acg    480
   Asp Thr Asn Met Gly Ala Trp Phe Glu Ala Gln Val Val Arg Val Thr
   145                 150                 155                 160
   cgg aag gcc ccc tcc cgg gac gag ccc tgc agc tcc acg tcc agg ccg    528
   Arg Lys Ala Pro Ser Arg Asp Glu Pro Cys Ser Ser Thr Ser Arg Pro
                   165                 170                 175
   gcg ctg gag gag gac gtc att tac cac gtg aaa tac gac gac tac ccg    576
   Ala Leu Glu Glu Asp Val Ile Tyr His Val Lys Tyr Asp Asp Tyr Pro
               180                 185                 190
   gag aac ggc gtg gtc cag atg aac tcc agg gac gtc cga gcg cgc gcc    624
   Glu Asn Gly Val Val Gln Met Asn Ser Arg Asp Val Arg Ala Arg Ala
           195                 200                 205
   cgc acc atc atc aag tgg cag gac ctg gag gtg ggc cag gtg gtc atg    672
   Arg Thr Ile Ile Lys Trp Gln Asp Leu Glu Val Gly Gln Val Val Met
       210                 215                 220
   ctc aac tac aac ccc gac aac ccc aag gag cgg ggc ttc tgg tac gac    720
   Leu Asn Tyr Asn Pro Asp Asn Pro Lys Glu Arg Gly Phe Trp Tyr Asp
   225                 230                 235                 240
   gcg gag atc tcc agg aag cgc gag acc agg acg gcg cgg gaa ctc tac    768
   Ala Glu Ile Ser Arg Lys Arg Glu Thr Arg Thr Ala Arg Glu Leu Tyr
                   245                 250                 255
   gcc aac gtg gtg ctg ggg gat gat tct ctg aac gac tgt cgg atc atc    816
   Ala Asn Val Val Leu Gly Asp Asp Ser Leu Asn Asp Cys Arg Ile Ile
               260                 265                 270
   ttc gtg gac gaa gtc ttc aag att gag cgg ccg ggt gaa ggg agc ccc    864
   Phe Val Asp Glu Val Phe Lys Ile Glu Arg Pro Gly Glu Gly Ser Pro
           275                 280                 285
   atg gtt gac aac ccc atg aga cgg aag agc ggg ccg tcc tgc aag cac    912
   Met Val Asp Asn Pro Met Arg Arg Lys Ser Gly Pro Ser Cys Lys His
       290                 295                 300
   tgc aag gac gac gtg aac aga ctc tgc agg gtc tgc gcc tgc cac ctg    960
   Cys Lys Asp Asp Val Asn Arg Leu Cys Arg Val Cys Ala Cys His Leu
   305                 310                 315                 320
   tgc ggg ggc cgg cag gac ccc gac aag cag ctc atg tgc gat gag tgc    1008
   Cys Gly Gly Arg Gln Asp Pro Asp Lys Gln Leu Met Cys Asp Glu Cys
                   325                 330                 335
   gac atg gcc ttc cac atc tac tgc ctg gac ccg ccc ctc agc agt gtt    1056
   Asp Met Ala Phe His Ile Tyr Cys Leu Asp Pro Pro Leu Ser Ser Val
               340                 345                 350
   ccc agc gag gac gag tgg tac tgc cct gag tgc cgg aat gat gcc agc    1104
   Pro Ser Glu Asp Glu Trp Tyr Cys Pro Glu Cys Arg Asn Asp Ala Ser
           355                 360                 365
   gag gtg gta ctg gcg gga gag cgg ctg aga gag agc aag aag aat gcg    1152
   Glu Val Val Leu Ala Gly Glu Arg Leu Arg Glu Ser Lys Lys Asn Ala
       370                 375                 380
   aag atg gcc tcg gcc aca tcg tcc tca cag cgg gac tgg ggc aag ggc    1200
   Lys Met Ala Ser Ala Thr Ser Ser Ser Gln Arg Asp Trp Gly Lys Gly
   385                 390                 395                 400
   atg gcc tgt gtg ggc cgc acc aag gaa tgt acc atc gtc ccg tcc aac    1248
   Met Ala Cys Val Gly Arg Thr Lys Glu Cys Thr Ile Val Pro Ser Asn
                   405                 410                 415
   cac tac gga ccc atc ccg ggg atc ccc gtg ggc acc atg tgg cgg ttc    1296
   His Tyr Gly Pro Ile Pro Gly Ile Pro Val Gly Thr Met Trp Arg Phe
               420                 425                 430
   cga gtc cag gtc agc gag tcg ggt gtc cat cgg ccc cac gtg gct ggc    1344
   Arg Val Gln Val Ser Glu Ser Gly Val His Arg Pro His Val Ala Gly
           435                 440                 445
   atc cat ggc cgg agc aac gac gga tcg tac tcc cta gtc ctg gcg ggg    1392
   Ile His Gly Arg Ser Asn Asp Gly Ser Tyr Ser Leu Val Leu Ala Gly
       450                 455                 460
   ggc tat gag gat gat gtg gac cat ggg aat ttt ttc aca tac acg ggt    1440
   Gly Tyr Glu Asp Asp Val Asp His Gly Asn Phe Phe Thr Tyr Thr Gly
   465                 470                 475                 480
   agt ggt ggt cga gat ctt tcc ggc aac aag agg acc gcg gaa cag tct    1488
   Ser Gly Gly Arg Asp Leu Ser Gly Asn Lys Arg Thr Ala Glu Gln Ser
                   485                 490                 495
   tgt gat cag aaa ctc acc aac acc aac agg gcg ctg gct ctc aac tgc    1536
   Cys Asp Gln Lys Leu Thr Asn Thr Asn Arg Ala Leu Ala Leu Asn Cys
               500                 505                 510
   ttt gct ccc atc aat gac caa gaa ggg gcc gag gcc aag gac tgg cgg    1584
   Phe Ala Pro Ile Asn Asp Gln Glu Gly Ala Glu Ala Lys Asp Trp Arg
           515                 520                 525
   tcg ggg aag ccg gtc agg gtg gtg cgc aat gtc aag ggt ggc aag aat    1632
   Ser Gly Lys Pro Val Arg Val Val Arg Asn Val Lys Gly Gly Lys Asn
       530                 535                 540
   agc aag tac gcc ccc gct gag ggc aac cgc tac gat ggc atc tac aag    1680
   Ser Lys Tyr Ala Pro Ala Glu Gly Asn Arg Tyr Asp Gly Ile Tyr Lys
   545                 550                 555                 560
   gtt gtg aaa tac tgg ccc gag aag ggg aag tcc ggg ttt ctc gtg tgg    1728
   Val Val Lys Tyr Trp Pro Glu Lys Gly Lys Ser Gly Phe Leu Val Trp
                   565                 570                 575
   cgc tac ctt ctg cgg agg gac gat gat gag cct ggc cct tgg acg aag    1776
   Arg Tyr Leu Leu Arg Arg Asp Asp Asp Glu Pro Gly Pro Trp Thr Lys
               580                 585                 590
   gag ggg aag gac cgg atc aag aag ctg ggg ctg acc atg cag tat cca    1824
   Glu Gly Lys Asp Arg Ile Lys Lys Leu Gly Leu Thr Met Gln Tyr Pro
           595                 600                 605
   gaa ggc tac ctg gaa gcc ctg gcc aac cga gag cga gag aag gag aac    1872
   Glu Gly Tyr Leu Glu Ala Leu Ala Asn Arg Glu Arg Glu Lys Glu Asn
       610                 615                 620
   agc aag agg gag gag gag gag cag cag gag ggg ggc ttc gcg tcc ccc    1920
   Ser Lys Arg Glu Glu Glu Glu Gln Gln Glu Gly Gly Phe Ala Ser Pro
   625                 630                 635                 640
   agg acg ggc aag ggc aag tgg aag cgg aag tcg gca gga ggt ggc ccg    1968
   Arg Thr Gly Lys Gly Lys Trp Lys Arg Lys Ser Ala Gly Gly Gly Pro
                   645                 650                 655
   agc agg gcc ggg tcc ccg cgc cgg aca tcc aag aaa acc aag gtg gag    2016
   Ser Arg Ala Gly Ser Pro Arg Arg Thr Ser Lys Lys Thr Lys Val Glu
               660                 665                 670
   ccc tac agt ctc acg gcc cag cag agc agc ctc atc aga gag gac aag    2064
   Pro Tyr Ser Leu Thr Ala Gln Gln Ser Ser Leu Ile Arg Glu Asp Lys
           675                 680                 685
   agc aac gcc aag ctg tgg aat gag gtc ctg gcg tca ctc aag gac cgg    2112
   Ser Asn Ala Lys Leu Trp Asn Glu Val Leu Ala Ser Leu Lys Asp Arg
       690                 695                 700
   ccg gcg agc ggc agc ccg ttc cag ttg ttc ctg agt aaa gtg gag gag    2160
   Pro Ala Ser Gly Ser Pro Phe Gln Leu Phe Leu Ser Lys Val Glu Glu
   705                 710                 715                 720
   acg ttc cag tgt atc tgc tgt cag gag ctg gtg ttc cgg ccc atc acg    2208
   Thr Phe Gln Cys Ile Cys Cys Gln Glu Leu Val Phe Arg Pro Ile Thr
                   725                 730                 735
   acc gtg tgc cag cac aac gtg tgc aag gac tgc ctg gac aga tcc ttt    2256
   Thr Val Cys Gln His Asn Val Cys Lys Asp Cys Leu Asp Arg Ser Phe
               740                 745                 750
   cgg gca cag gtg ttc agc tgc cct gcc tgc cgc tac gac ctg ggc cgc    2304
   Arg Ala Gln Val Phe Ser Cys Pro Ala Cys Arg Tyr Asp Leu Gly Arg
           755                 760                 765
   agc tat gcc atg cag gtg aac cag cct ctg cag acc gtc ctc aac cag    2352
   Ser Tyr Ala Met Gln Val Asn Gln Pro Leu Gln Thr Val Leu Asn Gln
       770                 775                 780
   ctc ttc ccc ggc tac ggc aat ggc cgg tga                            2382
   Leu Phe Pro Gly Tyr Gly Asn Gly Arg
   785                 790
<210> 2
   <211> 793
   <212> PRT
   <213> Homo sapiens
<400> 2
   Met Trp Ile Gln Val Arg Thr Met Asp Gly Arg Gln Thr His Thr Val
    1               5                  10                  15
   Asp Ser Leu Ser Arg Leu Thr Lys Val Glu Glu Leu Arg Arg Lys Ile
               20                  25                  30
   Gln Glu Leu Phe His Val Glu Pro Gly Leu Gln Arg Leu Phe Tyr Arg
           35                  40                  45
   Gly Lys Gln Met Glu Asp Gly His Thr Leu Phe Asp Tyr Glu Val Arg
   50 55 60
   Leu Asn Asp Thr Ile Gln Leu Leu Val Arg Gln Ser Leu Val Leu Pro
   65 70 75 80
   His Ser Thr Lys Glu Arg Asp Ser Glu Leu Ser Asp Thr Asp Ser Gly
   85 90 95
   Cys Cys Leu Gly Gln Ser Glu Ser Asp Lys Ser Ser Thr His Gly Glu
   100 105 110
   Ala Ala Ala Glu Thr Asp Ser Arg Pro Ala Asp Glu Asp Met Trp Asp
   115 120 125
   Glu Thr Glu Leu Gly Leu Tyr Lys Val Asn Glu Tyr Val Asp Ala Arg
   130 135 140
   Asp Thr Asn Met Gly Ala Trp Phe Glu Ala Gln Val Val Arg Val Thr
   145 150 155 160
   Arg Lys Ala Pro Ser Arg Asp Glu Pro Cys Ser Ser Thr Ser Arg Pro
   165 170 175
   Ala Leu Glu Glu Asp Val Ile Tyr His Val Lys Tyr Asp Asp Tyr Pro
   180 185 190
   Glu Asn Gly Val Val Gln Met Asn Ser Arg Asp Val Arg Ala Arg Ala
   195 200 205
   Arg Thr Ile Ile Lys Trp Gln Asp Leu Glu Val Gly Gln Val Val Met
   210 215 220
   Leu Asn Tyr Asn Pro Asp Asn Pro Lys Glu Arg Gly Phe Trp Tyr Asp
   225 230 235 240
   Ala Glu Ile Ser Arg Lys Arg Glu Thr Arg Thr Ala Arg Glu Leu Tyr
   245 250 255
   Ala Asn Val Val Leu Gly Asp Asp Ser Leu Asn Asp Cys Arg Ile Ile
   260 265 270
   Phe Val Asp Glu Val Phe Lys Ile Glu Arg Pro Gly Glu Gly Ser Pro
   275 280 285
   Met Val Asp Asn Pro Met Arg Arg Lys Ser Gly Pro Ser Cys Lys His
   290 295 300
   Cys Lys Asp Asp Val Asn Arg Leu Cys Arg Val Cys Ala Cys His Leu
   305 310 315 320
   Cys Gly Gly Arg Gln Asp Pro Asp Lys Gln Leu Met Cys Asp Glu Cys
   325 330 335
   Asp Met Ala Phe His Ile Tyr Cys Leu Asp Pro Pro Leu Ser Ser Val
   340 345 350
   Pro Ser Glu Asp Glu Trp Tyr Cys Pro Glu Cys Arg Asn Asp Ala Ser
   355 360 365
   Glu Val Val Leu Ala Gly Glu Arg Leu Arg Glu Ser Lys Lys Asn Ala
   370 375 380
   Lys Met Ala Ser Ala Thr Ser Ser Ser Gln Arg Asp Trp Gly Lys Gly
   385 390 395 400
   Met Ala Cys Val Gly Arg Thr Lys Glu Cys Thr Ile Val Pro Ser Asn
   405 410 415
   His Tyr Gly Pro Ile Pro Gly Ile Pro Val Gly Thr Met Trp Arg Phe
   420 425 430
   Arg Val Gln Val Ser Glu Ser Gly Val His Arg Pro His Val Ala Gly
   435 440 445
   Ile His Gly Arg Ser Asn Asp Gly Ser Tyr Ser Leu Val Leu Ala Gly
   450 455 460
   Gly Tyr Glu Asp Asp Val Asp His Gly Asn Phe Phe Thr Tyr Thr Gly
   465 470 475 480
   Ser Gly Gly Arg Asp Leu Ser Gly Asn Lys Arg Thr Ala Glu Gln Ser
   485 490 495
   Cys Asp Gln Lys Leu Thr Asn Thr Asn Arg Ala Leu Ala Leu Asn Cys
   500 505 510
   Phe Ala Pro Ile Asn Asp Gln Glu Gly Ala Glu Ala Lys Asp Trp Arg
   515 520 525
   Ser Gly Lys Pro Val Arg Val Val Arg Asn Val Lys Gly Gly Lys Asn
   530 535 540
   Ser Lys Tyr Ala Pro Ala Glu Gly Asn Arg Tyr Asp Gly Ile Tyr Lys
   545 550 555 560
   Val Val Lys Tyr Trp Pro Glu Lys Gly Lys Ser Gly Phe Leu Val Trp
   565 570 575
   Arg Tyr Leu Leu Arg Arg Asp Asp Asp Glu Pro Gly Pro Trp Thr Lys
   580 585 590
   Glu Gly Lys Asp Arg Ile Lys Lys Leu Gly Leu Thr Met Gln Tyr Pro
   595 600 605
   Glu Gly Tyr Leu Glu Ala Leu Ala Asn Arg Glu Arg Glu Lys Glu Asn
   610 615 620
   Ser Lys Arg Glu Glu Glu Glu Gln Gln Glu Gly Gly Phe Ala Ser Pro
   625 630 635 640
   Arg Thr Gly Lys Gly Lys Trp Lys Arg Lys Ser Ala Gly Gly Gly Pro
   645 650 655
   Ser Arg Ala Gly Ser Pro Arg Arg Thr Ser Lys Lys Thr Lys Val Glu
   660 665 670
   Pro Tyr Ser Leu Thr Ala Gln Gln Ser Ser Leu Ile Arg Glu Asp Lys
   675 680 685
   Ser Asn Ala Lys Leu Trp Asn Glu Val Leu Ala Ser Leu Lys Asp Arg
   690 695 700
   Pro Ala Ser Gly Ser Pro Phe Gln Leu Phe Leu Ser Lys Val Glu Glu
   705                 710                 715                 720
   Thr Phe Gln Cys Ile Cys Cys Gln Glu Leu Val Phe Arg Pro Ile Thr
                   725                 730                 735
   Thr Val Cys Gln His Asn Val Cys Lys Asp Cys Leu Asp Arg Ser Phe
               740                 745                 750
   Arg Ala Gln Val Phe Ser Cys Pro Ala Cys Arg Tyr Asp Leu Gly Arg
           755                 760                 765
   Ser Tyr Ala Met Gln Val Asn Gln Pro Leu Gln Thr Val Leu Asn Gln
       770                 775                 780
   Leu Phe Pro Gly Tyr Gly Asn Gly Arg
   785                 790
<210> 3
   <211> 45
   <212> ADN
   <213> Homo sapiens
<220>
   <221> CDS
   <222> (1)..(45)
<400> 3
   acc cac ggt gag gcg gcc gcc gag act gac agc agg cca gcc gat       45
   Thr His Gly Glu Ala Ala Ala Glu Thr Asp Ser Arg Pro Ala Asp
     1               5                  10                  15
<210> 4
   <211> 15
   <212> PRT
   <213> Homo sapiens
<400> 4
   Thr His Gly Glu Ala Ala Ala Glu Thr Asp Ser Arg Pro Ala Asp
     1               5                  10                  15
<210> 5
   <211> 78
   <212> ADN
   <213> Homo sapiens
<220>
   <221> CDS
   <222> ,(1)..(78)
<400> 5
   atg gtt gac aac ccc atg aga cgg aag agc ggg ccg tcc tgc aag cac     48
   Met Val Asp Asn Pro Met Arg Arg Lys Ser Gly Pro Ser Cys Lys His
     1               5                  10                  15
   tgc aag gac gac gtg aac aga ctc tgc agc                             78
                   Cys Lys Asp Asp Val Asn Arg Leu Cys Ser
                               20                  25
<210> 6
   <211> 26
   <212> PRT
   <213> Homo sapiens
<400> 6
   Met Val Asp Asn Pro Met Arg Arg Lys Ser Gly Pro Ser Cys Lys His
     1               5                  10                  15
   Cys Lys Asp Asp Val Asn Arg Leu Cys Ser
               20                  25
<210> 7
   <211> 525
   <212> ADN
   <213> Homo sapiens
<220>
   <221> CDS
   <222> (1)..(525)
<400> 7
   cga gag aag gag aac agc aag agg gag gag gag gag cag cag gag ggg     48
   Arg Glu Lys Glu Asn Ser Lys Arg Glu Glu Glu Glu Gln Gln Glu Gly
    1               5                  10                  15
   ggc ttc gcg tcc ccc agg acg ggc aag ggc aag tgg aag cgg aag tcg     96
   Gly Phe Ala Ser Pro Arg Thr Gly Lys Gly Lys Trp Lys Arg Lys Ser
               20                  25                  30
   gca gga ggt ggc ccg agc agg gcc ggg tcc ccg cgc cgg aca tcc aag    144
   Ala Gly Gly Gly Pro Ser Arg Ala Gly Ser Pro Arg Arg Thr Ser Lys
           35                  40                  45
   aaa acc aag gtg gag ccc tac agt ctc acg gcc cag cag agc agc ctc    192
   Lys Thr Lys Val Glu Pro Tyr Ser Leu Thr Ala Gln Gln Ser Ser Leu
       50                  55                  60
   atc aga gag gac aag agc aac gcc aag ctg tgg aat gag gtc ctg gcg    240
   Ile Arg Glu Asp Lys Ser Asn Ala Lys Leu Trp Asn Glu Val Leu Ala
   65                  70                  75                  80
   tca ctc aag gac cgg ccg gcg agc ggc agc ccg ttc cag ttg ttc ctg    288
   Ser Leu Lys Asp Arg Pro Ala Ser Gly Ser Pro Phe Gln Leu Phe Leu
                   85                  90                  95
   agt aaa gtg gag gag acg ttc cag tgt atc tgc tgt cag gag ctg gtg    336
   Ser Lys Val Glu Glu Thr Phe Gln Cys Ile Cys Cys Gln Glu Leu Val
               100                 105                 110
   ttc cgg ccc atc acg acc gtg tgc cag cac aac gtg tgc aag gac tgc    384
   Phe Arg Pro Ile Thr Thr Val Cys Gln His Asn Val Cys Lys Asp Cys
           115                 120                 125
   ctg gac aga tcc ttt cgg gca cag gtg ttc agc tgc cct gcc tgc cgc    432
   Leu Asp Arg Ser Phe Arg Ala Gln Val Phe Ser Cys Pro Ala Cys Arg
       130                 135                 140
   tac gac ctg ggc cgc agc tat gcc atg cag gtg aac cag cct ctg cag    480
   Tyr Asp Leu Gly Arg Ser Tyr Ala Met Gln Val Asn Gln Pro Leu Gln
   145                 150                 155                 160
   acc gtc ctc aac cag ctc ttc ccc ggc tac ggc aat ggc cgg tga        525
   Thr Val Leu Asn Gln Leu Phe Pro Gly Tyr Gly Asn Gly Arg
                   165                 170                 175
<210> 8
   <211> 174
   <212> PRT
   <213> Homo sapiens
<400> 8
   Arg Glu Lys Glu Asn Ser Lys Arg Glu Glu Glu Glu Gln Gln Glu Gly
   1               5                   10                  15
   Gly Phe Ala Ser Pro Arg Thr Gly Lys Gly Lys Trp Lys Arg Lys Ser
               20                  25                  30
   Ala Gly Gly Gly Pro Ser Arg Ala Gly Ser Pro Arg Arg Thr Ser Lys
           35                  40                  45
   Lys Thr Lys Val Glu Pro Tyr Ser Leu Thr Ala Gln Gln Ser Ser Leu
       50                  55                  60
   Ile Arg Glu Asp Lys Ser Asn Ala Lys Leu Trp Asn Glu Val Leu Ala
   65                  70                  75                      80
   Ser Leu Lys Asp Arg Pro Ala Ser Gly Ser Pro Phe Gln Leu Phe Leu
                   85                  90                  95
   Ser Lys Val Glu Glu Thr Phe Gln Cys Ile Cys Cys Gln Glu Leu Val
               100                 105                 110
   Phe Arg Pro Ile Thr Thr Val Cys Gln His Asn Val Cys Lys Asp Cys
           115                 120                 125
   Leu Asp Arg Ser Phe Arg Ala Gln Val Phe Ser Cys Pro Ala Cys Arg
       130                 135                 140
   Tyr Asp Leu Gly Arg Ser Tyr Ala Met Gln Val Asn Gln Pro Leu Gln
   145                 150                 155                 160
   Thr Val Leu Asn Gln Leu Phe Pro Gly Tyr Gly Asn Gly Arg
                   165                 170
<210> 9
   <211> 324
   <212> ADN
   <213> Homo sapiens
<400> 9
   atgtggatcc aggttcggac catggatggg aggcagaccc acacggtgga ctcgctgtcc 60
   aggctgacca aggtggagga gctgaggcgg aagatccagg agctgttcca cgtggagcca 120
   ggcctgcaga ggctgttcta caggggcaaa cagatggagg acggccatac cctcttcgac 180
   tacgaggtcc gcctgaatga caccatccag ctcctggtcc gccagagcct cgtgctcccc 240
   cacagcacca aggagcggga ctccgagctc tccgacaccg actccggctg ctgcctgggc 300
   cagagtgagt cagacaagtc ctcc                                        324
<210> 10
   <211> 495
   <212> ADN
   <213> Homo sapiens
<400> 10
   gaggacatgt gggatgagac ggaattgggg ctgtacaagg tcaatgagta cgtcgatgct 60
   cgggacacga acatgggggc gtggtttgag gcgcaggtgg tcagggtgac gcggaaggcc 120
   ccctcccggg acgagccctg cagctccacg tccaggccgg cgctggagga ggacgtcatt 180
   taccacgtga aatacgacga ctacccggag aacggcgtgg tccagatgaa ctccagggac 240
   gtccgagcgc gcgcccgcac catcatcaag tggcaggacc tggaggtggg ccaggtggtc 300
   atgctcaact acaaccccga caaccccaag gagcggggct tctggtacga cgcggagatc 360
   tccaggaagc gcgagaccag gacggcgcgg gaactctacg ccaacgtggt gctgggggat 420
   gattctctga acgactgtcg gatcatcttc gtggacgaag tcttcaagat tgagcggccg 480
   ggtgaaggga gcccc 495
<210> 11
   <211> 915
   <212> ADN
   <213> Homo sapiens
<400> 11
   gtctgcgcct gccacctgtg cgggggccgg caggaccccg acaagcagct catgtgcgat 60
   gagtgcgaca tggccttcca catctactgc ctggacccgc ccctcagcag tgttcccagc 120
   gaggacgagt ggtactgccc tgagtgccgg aatgatgcca gcgaggtggt actggcggga 180
   gagcggctga gagagagcaa gaagaatgcg aagatggcct cggccacatc gtcctcacag 240
   cgggactggg gcaagggcat ggcctgtgtg ggccgcacca aggaatgtac catcgtcccg 300
   tccaaccact acggacccat cccggggatc cccgtgggca ccatgtggcg gttccgagtc 360
   caggtcagcg agtcgggtgt ccatcggccc cacgtggctg gcatccatgg ccggagcaac 420
   gacggatcgt actccctagt cctggcgggg ggctatgagg atgatgtgga ccatgggaat 480
   tttttcacat acacgggtag tggtggtcga gatctttccg gcaacaagag gaccgcggaa 540
   cagtcttgtg atcagaaact caccaacacc aacagggcgc tggctctcaa ctgctttgct 600
   cccatcaatg accaagaagg ggccgaggcc aaggactggc ggtcggggaa gccggtcagg 660
   gtggtgcgca atgtcaaggg tggcaagaat agcaagtacg cccccgctga gggcaaccgc 720
   tacgatggca tctacaaggt tgtgaaatac tggcccgaga aggggaagtc cgggtttctc 780
   gtgtggcgct accttctgcg gagggacgat gatgagcctg gcccttggac gaaggagggg 840
   aaggaccgga tcaagaagct ggggctgacc atgcagtatc cagaaggcta cctggaagcc 900
   ctggccaacc gagag 915
<210> 12
   <211> 1366
   <212> ADN
   <213> Homo sapiens
<400> 12
   ggcagcgttt gccgagcggg cgctccgggt cgcacgcaag tccgcgcggg gtccgggcca 60
   cgcacgcggt ttcatcgcca tccccagccg ggccaggcgc gcaggcagac aagctgttcg 120
   cggcgaccgg agaggtgagc gggcgggccg ggtcggggtg ccagcccggg ccgggcgcac 180
   ggggctcggg aactttgcaa aactttcccg cgcggccagc ccgggcgcac gcatgtcccg 240
   cactctgtcc cgggatccag ggcctcccct tccacctaac cctcgggaat cgttccccgg 300
   cacacatccg gctggagccg ggaccagcgc tgcgtccccg gagcccggcg gggggtcgag 360
   cgcgccgggt gggggagggc ctggcgagcc gccggggagg atgtcaggct ccgcgcctgc 420
   gcgcggggcg ccccgcgatt caattgtcgc gcccgagccc gatttcgcgc gccctgagtt 480
   ccccgggagc atctgggcca atggggagcg agcggggcgg ggcggccggg tgctgcggag 540
   ccaataagag gcggctcaag tgaagggggg cgggacttga cgagcggggg ccccctctgt 600
   agtcccggcg gcgggggtgg gcgtgggctc gctggcgcga cccgcgcggg ccagtgggag 660
   tgcgggaggg acgccgaggg tccagggttt ggaggggcgc gagctgccgg gggttggagg 720
   tcgaggtgag tcgcggggcg cgcgcgctcg cgggtggccg ggacggggcg cggttaccat 780
   ggccaccgcg gggcgggccc ggtcgcgcac gcgcgcgggg ggggccggca aggagggggg 840
   gcgtgggcac cgaggggtcc cggggtccgc ggatctcggg tggggttttt cccatttcag 900
   tggcacttgg ttaagttccc ccgggacctt ctgaagttcc ggcccgcgct ggactttctg 960
   ggattccctc ttccgtaaat aggaatccga ggaatgaatg aatcaatgaa tgaatgaata 1020
   aacgaaccaa ctcgggccac ttggcccggg cctcctttct cctctggtcg tggggaagga 1080
   gggatgggtt ggaccttctg cttttctttc aattccctct tttcattctc cttcctcctc 1140
   aatcttcaac acttggctag tcgttaatgc cttaagtgct taatttgttg tgtctggtcc 1200
   tggccagggt ctggctgtac aggaggactg gaagggcatc ctgggagttt cctggtgtcc 1260
   acaggccgga caaaagcaac cccgactcct tagagcatgg catggctcag aggtgctggt 1320
   aaaactgatg ggggtttatg ctgtccctcc cctcagcgcc gacacc 1366